# EUROPÄISCHE PATENTSCHRIFT

(11) **EP 0 903 339 B1**
(45) Veröffentlichungstag und Bekanntmachung des Hinweises auf die Patenterteilung: **12.12.2001**
(21) Anmeldenummer: 98117529.2
(22) Anmeldetag: 16.09.1998
(51) Int. Cl.: C07C 311/16, A61K 31/275

(54) **Biphenylsulfonylcyanamide, Verfahren zu ihrer Herstellung und ihre Verwendung als Medikament**
Biphenylsulfonylcyanamides, process for their preparation and their use as medicaments
Biphénylsulfonylcyanamides, procédé pour leur préparation et leur utilisation comme medicaments

(30) Priorität: 22.09.1997 DE 19741635
(43) Veröffentlichungstag der Anmeldung: 24.03.1999
(73) Patentinhaber: Aventis Pharma Deutschland GmbH, 65929 Frankfurt am Main (DE)
(72) Erfinder: Kleemann, Heinz-Werner, Dr., 65474 Bischofsheim (DE); Schwark, Jan-Robert, Dr., 65799 Kelkheim (DE); Faber, Sabine, Dr., 65510 Idstein (DE); Lang, Hans Jochen, Dr., 65719 Hofheim (DE); Weichert, Andreas, Dr., 63329 Egelsbach (DE); Jansen, Hans-Willi, Dr., 65527 Niedernhausen (DE)

(56) Entgegenhaltungen:
- US-A- 5 281 614
- US-A- 5 412 097

## Beschreibung

Die Erfindung betrifft Verbindungen der Formel (I), 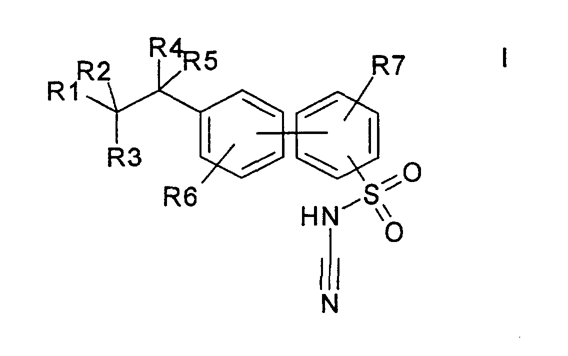 in welcher die Symbole folgende Bedeutung haben:
- R(1): Wasserstoff, Alkyl mit 1,2,3,4,5,6,7 oder 8 C-Atomen, 1-Naphthyl, 2-Naphthyl, -CₐH₂ₐ-Cycloalkyl mit 3,4,5,6 oder 7 C-Atomen oder -CₐH₂ₐ-Phenyl, wobei der Phenylteil unsubstituiert oder substituiert ist mit 1-3 Substituenten aus der Gruppe Alkyl mit 1,2,3,4,5,6,7 oder 8 C-Atomen, F, Cl, Br, I, CF₃, SOₙR(11), OR(17), NR(8)R(9), -C≡N, -NO₂ oder CO-R(22);
R(11) Alkyl mit 1,2,3 oder 4 C-Atomen oder NR(20)R(21);
R(20) und R(21) unabhängig voneinander Wasserstoff oder Alkyl mit 1,2,3 oder 4 C-Atomen;
R(17) Wasserstoff oder Alkyl mit 1,2,3 oder 4 C-Atomen;
R(8) und R(9) unabhängig voneinander Wasserstoff oder Alkyl mit 1,2,3 oder 4 C-Atomen;
R(22) Wasserstoff, Alkyl mit 1,2,3,4,5,6,7 oder 8 C-Atomen oder OR(30);
R(30) Wasserstoff, Alkyl mit 1,2,3,4,5,6,7 oder 8 C-Atomen;
a Null, 1 oder 2;
n Null, 1 oder 2;
oder
- R(1) und R(3): gemeinsam mit dem sie tragenden C-Atom Cycloalkyl mit 3, 4, 5, 6 oder 7 C-Atomen oder Fluorenyl;
- R(2), R(3), R(4) und R(5): unabhängig voneinander Wasserstoff, F, CF₃, O-R(10), Alkyl mit 1,2,3,4,5,6,7 oder 8 C-Atomen, Cycloalkyl mit 3, 4, 5, 6 oder 7 C-Atomen, -C_{g}H_{2g}-Phenyl, wobei der Phenylteil unsubstituiert oder substituiert ist mit 1-3 Substituenten aus der Gruppe F, Cl, Br, I, CF₃, Methyl, Methoxy, Hydroxy oder NR(18)R(19);
R(18) und R(19) unabhängig voneinander Wasserstoff oder Alkyl mit 1,2,3 oder 4 C-Atomen;
g Null, 1 oder 2;
R(10) Wasserstoff, Alkyl mit 1,2,3,4,5,6,7 oder 8 C-Atomen, Phenyl, das unsubstituiert oder substituiert ist mit 1-3 Substituenten aus der Gruppe F, Cl, Br, I, CF₃, Methyl, Methoxy, Hydroxy oder NR(12)R(13);
R(12) und R(13) unabhängig voneinander Wasserstoff oder Alkyl mit 1,2,3 oder 4 C-Atomen;
oder
R(10) Heteroaryl mit 1,2,3,4,5,6,7,8 oder 9-C-Atomen, das unsubstituiert oder substituiert ist mit 1-3 Substituenten aus der Gruppe F, Cl, Br, I, CF₃, CH₃, Methoxy, Hydroxy oder NR(14)R(15);
R(14) und R(15) unabhängig voneinander Wasserstoff oder Alkyl mit 1,2,3 oder 4 C-Atomen; oder

- R(2) und R(4): gemeinsam eine zweite Bindung zwischen den die Reste R(3) und R(5) tragenden Kohlenstoffatomen, wobei R(1), R(3), R(5) wie oben definiert sind;
- R(6) und R(7): unabhängig voneinander Wasserstoff, F, Cl, Br, I, CF₃, -C≡N, -NO₂, SOₚ-R(16),CO-R(23) oder O-R(24);
R(23) Wasserstoff, Alkyl mit 1,2,3,4,5,6,7 oder 8 C-Atomen oder OR(25);
R(25) Wasserstoff, Alkyl mit 1,2,3,4,5,6,7 oder 8 C-Atomen;
R(24) Wasserstoff, Alkyl mit 1,2,3,4,5,6,7 oder 8 C-Atomen oder Phenyl, das unsubstituiert oder substituiert ist mit 1-3 Substituenten aus der Gruppe F, Cl, Br, I, CF₃, Methyl, Methoxy, Hydroxy oder NR(28)R(29);
R(28) und R(29) H oder Alkyl mit 1,2,3 oder 4 C-Atomen;
R(16) Alkyl mit 1,2,3,4,5,6,7 oder 8 C-Atomen, Phenyl, das unsubstituiert oder substituiert ist mit 1-3 Substituenten aus der Gruppe F, Cl, Br, I, CF₃, Methyl, Methoxy, Hydroxy oder NR(26)R(27);
R(26) und R(27) gleich H oder Alkyl mit 1,2,3 oder 4 C-Atomen;
p Null, 1 oder 2;
sowie deren physiologisch verträglichen Salze.

Bevorzugt sind Verbindungen der Formel (I), mit
- R(1): Wasserstoff, Alkyl mit 1,2,3 oder 4 C-Atomen, 1-Naphthyl, 2-Naphthyl, -CₐH₂ₐ-Cycloalkyl mit 3, 4, 5, 6 oder 7 C-Atomen oder -CₐH₂ₐ-Phenyl, wobei der Phenylteil unsubstituiert oder substituiert ist mit 1-2 Substituenten aus der Gruppe Alkyl mit 1,2,3 oder 4 C-Atomen, F, Cl, CF₃, SOₙR(11), OR(17), NR(8)R(9), -C≡N, oder CO-R(22);
R(11) Alkyl mit 1,2,3 oder 4 C-Atomen oder NR(20)R(21);
R(20) und R(21) unabhängig voneinander Wasserstoff, Methyl oder Ethyl;
R(17) Wasserstoff oder Alkyl mit 1,2,3 oder 4 C-Atomen;
R(8) und R(9) unabhängig voneinander Wasserstoff, Methyl oder Ethyl;
R(22) Wasserstoff, Alkyl mit 1,2,3 oder 4 C-Atomen oder OR(30);
R(30) Wasserstoff, Alkyl mit 1,2,3 oder 4 C-Atomen;
a Null oder 1;
n Null oder 2;
oder
- R(1) und R(3): gemeinsam mit dem sie tragenden C-Atom Cycloalkyl mit 3, 4, 5, 6 oder 7 C-Atomen oder Fluorenyl;
- R(2) und R(4): unabhängig voneinander Wasserstoff oder F; oder
- R(2) und R(4): gemeinsam eine zweite Bindung zwischen den die Reste R(3) und R(5) tragenden Kohlenstoffatomen;
- R(3) und R(5): unabhängig voneinander Wasserstoff, F, CF₃, O-R(10), Alkyl mit 1,2,3 oder 4 C-Atomen, Cycloalkyl mit 3, 4, 5, 6 oder 7 C-Atomen, -C_{g}H_{2g}-Phenyl, wobei der Phenylteil unsubstituiert oder substituiert ist mit 1-2 Substituenten aus der Gruppe F, Cl, CF₃, Methyl, Methoxy, Hydroxy oder NR(18)R(19);
R(18) und R(19) unabhängig voneinander gleich Wasserstoff, Methyl oder Ethyl;
g Null oder 1 ;
R(10) Wasserstoff, Alkyl mit 1, 2, 3 oder 4 C-Atomen, Phenyl, das unsubstituiert oder substituiert ist mit 1-2 Substituenten aus der Gruppe F, Cl, CF₃, Methyl, Methoxy, Hydroxy oder NR(12)R(13);
R(12) und R(13) unabhängig voneinander gleich Wasserstoff, Methyl oder Ethyl; oder
R(10) Heteroaryl mit 1, 2, 3, 4, 5, 6, 7, 8 oder 9 C-Atomen, das unsubstituiert oder substituiert ist mit 1-2 Substituenten aus der Gruppe F, Cl, CF₃, CH₃, Methoxy, Hydroxy oder NR(14)R(15);
R(14) und R(15) unabhängig voneinander Wasserstoff, Methyl oder Ethyl;
- R(6) und R(7): unabhängig voneinander Wasserstoff, F, Cl, CF₃, -C≡N, SOₚ-R(16), CO-R(23) oder O-R(24);
R(23) Wasserstoff, Alkyl mit 1,2,3 oder 4 C-Atomen oder OR(25);
R(25) Wasserstoff, Alkyl mit 1,2,3 oder 4 C-Atomen;
R(24) Wasserstoff, Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(28)R(29);
R(28) und R(29) unabhängig voneinander Wasserstoff, Methyl oder Ethyl;
R(16) Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(26)R(27);
R(26) und R(27) unabhängig voneinander Wasserstoff, Methyl oder Ethyl;
p Null oder 2;
sowie deren physiologisch verträglichen Salze.

Besonders bevorzugt sind Verbindungen der Formel (I), mit
- R(1): Methyl, Ethyl, 1-Naphthyl, 2-Naphthyl,-CₐH₂ₐ-Cycloalkyl mit 3, 4, 5, 6 oder 7 C-Atomen oder -CₐH₂ₐ-Phenyl, wobei der Phenylteil unsubstituiert oder substituiert ist mit 1-2 Substituenten aus der Gruppe Alkyl mit 1,2,3 oder 4 C-Atomen, F, Cl, CF₃, SO₂R(11), OR(17), NR(8)R(9), -C≡N, oder CO-R(22);
R(11) Methyl oder Dimethylamino;
R(17) Wasserstoff, Methyl oder Ethyl;
R(8) und R(9) unabhängig voneinander Wasserstoff, Methyl oder Ethyl,
R(22) Wasserstoff oder Alkyl mit 1,2,3 oder 4 C-Atomen;
a Null oder 1;
oder
- R(1) und R(3): gemeinsam mit dem sie tragenden C-Atom Cycloalkyl mit 3,4, 5, 6 oder 7 C-Atomen oder Fluorenyl;
- R(2) und R(4): unabhängig voneinander Wasserstoff oder F; oder
- R(2) und R(4): gemeinsam eine zweite Bindung zwischen den die Reste R(3) und R(5) tragenden Kohlenstoffatomen;
- R(3) und R(5): unabhängig voneinander Wasserstoff, F, CF₃, O-R(10), Alkyl mit 1,2,3 oder 4 C-Atomen oder -C_{g}H_{2g}-Phenyl, das unsubstituiert oder substituiert ist mit 1-2 Substituenten aus der Gruppe F, Cl, CF₃, Methyl, Methoxy, Hydroxy oder NR(18)R(19);
R(18) und R(19) unabhängig voneinander Wasserstoff, Methyl oder Ethyl;
g Null oder 1;
R(10) Wasserstoff, Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(12)R(13);
R(12) und R(13) gleich Wasserstoff, Methyl oder Ethyl; oder
R(10) Heteroaryl mit 1,2,3,4,5,6,7,8 oder 9 C-Atomen, das unsubstituiert oder substituiert ist mit 1-2 Substituenten aus der Gruppe F, Cl, CF₃, CH₃, Methoxy, Hydroxy oder Dimethylamino;
- R(6) und R(7): unabhängig voneinander Wasserstoff, F, Cl, CF₃, -C≡N, SO₂-R(16), CO-R(23) oder O-R(24);
R(23) Wasserstoff oder Alkyl mit 1, 2, 3 oder 4 C-Atomen;
R(24) Wasserstoff, Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(28)R(29);
R(28) und R(29) unabhängig voneinander Wasserstoff, Methyl oder Ethyl;
R(16) Alkyl mit 1,2,3 oder 4 C-Atomen;
sowie deren physiologisch verträglichen Salze.

Ganz besonders bevorzugt sind Verbindungen der Formel (I), mit
- R(1): Methyl, Ethyl, 1-Naphthyl, 2-Naphthyl, Cycloalkyl mit 3, 4, 5, 6 oder 7 C-Atomen oder Phenyl, das unsubstituiert oder substituiert ist mit einem Substituenten aus der Gruppe Alkyl mit 1,2,3 oder 4 C-Atomen, F, Cl, CF₃, SO₂R(11), OR(17), NR(8)R(9), oder CO-R(22);
R(11) Methyl oder Dimethylamino;
R(17) Wasserstoff, Methyl oder Ethyl;
R(8) und R(9) unabhängig voneinander gleich Wasserstoff, Methyl oder Ethyl;
R(22) Wasserstoff oder Alkyl mit 1,2,3 oder 4 C-Atomen;
oder
- R(1) und R(3): gemeinsam mit dem sie tragenden C-Atom Cycloalkyl mit 3, 4, 5, 6 oder 7 C-Atomen oder Fluorenyl;
- R(2) und R(4): Wasserstoff; oder
- R(2) und R(4): gemeinsam eine zweite Bindung zwischen den die Reste R(3) und R(5) tragenden Kohlenstoffatomen;
- R(3) und R(5): unabhängig voneinander Wasserstoff, CF₃, O-R(10), Alkyl mit 1, 2, 3 oder 4 C-Atomen oder Phenyl, das unsubstituiert oder substituiert ist mit einem Substituenten aus der Gruppe F, Cl, CF₃, Methyl, Methoxy, Hydroxy oder NR(18)R(19);
R(18) und R(19) unabhängig voneinander Wasserstoff, Methyl oder Ethyl;
R(10) Wasserstoff, Alkyl mit 1,2,3 oder 4 C-Atomen oder Phenyl, das unsubstituiert oder substituiert ist mit 1 Substituenten aus der Gruppe F, Cl, CF₃, Methyl, Methoxy, Hydroxy oder NR(12)R(13);
R(12) und R(13) unabhängig voneinander Wasserstoff, Methyl oder Ethyl, oder
R(10) Heteroaryl mit 1, 2, 3, 4, 5, 6, 7, 8 oder 9 C-Atomen, das unsubstituiert oder substituiert ist mit einem Substituenten aus der Gruppe F, Cl, CF₃, CH₃, Methoxy, Hydroxy oder Dimethylamino;
- R(6) und R(7): unabhängig voneinander Wasserstoff, F, Cl, CF₃, SO₂-CH₃, CO-R(23) oder O-R(24);
R(23) Wasserstoff oder Alkyl mit 1,2,3 oder 4 C-Atomen;
R(24) Wasserstoff, Alkyl mit 1,2,3 oder 4 C-Atomen oder Phenyl, das unsubstituiert oder substituiert ist mit 1 Substituenten aus der Gruppe F, Cl, CF₃, Methyl, Methoxy, Hydroxy oder NR(28)R(29);
R(28) und R(29) unabhängig voneinander Wasserstoff, Methyl oder Ethyl;
sowie deren physiologisch verträglichen Salze.

Bevorzugt sind auch Verbindungen der Formel I, in denen der Biphenylring wie in Verbindungen der Formel 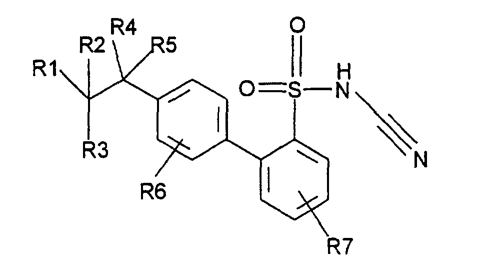 verknüpft ist und die Sulfonylcyanamidgruppe sich an der 2 Position befindet.

Bevorzugt sind ferner Verbindungen der Formel I, in denen R(3) und R(5) Wasserstoff und R(2) und R(4) ebenfalls Wasserstoff oder gemeinsam eine Bindung bedeuten.

Ferner sind Verbindungen der Formel I bevorzugt, in denen R(1) und R(3) zusammen mit dem sie tragenden C-Atom Cycloalkyl mit 3,4,5,6 oder 7 C-Atomen bedeuten.

Bevorzugt sind auch Verbindungen der Formel I in den R(1) Phenyl ist, das vorzugsweise unsubstituiert ist oder substituiert ist mit einem Substituenten aus der Gruppe Alkyl mit 1,2,3 oder 4 C-Atomen, F, Cl, CF₃, SO₂R(11), OR(17), NR(8)R(9), oder CO-R(22);
- R(11): Methyl oder Dimethylamino;
- R(17): Wasserstoff, Methyl oder Ethyl;
- R(8) und R(9): unabhängig voneinander gleich Wasserstoff, Methyl oder Ethyl;
- R(22): Wasserstoff oder Alkyl mit 1,2,3 oder 4 C-Atomen.

Bevorzugt sind auch Verbindungen der Formel I, in denen R(6) und R(7) Wasserstoff sind.

Alkyl kann geradkettig oder verzweigt sein.

Unter Cycloalkyl werden auch Alkyl-substituierte Ringe verstanden.

Beipiele für Alkylreste mit 1, 2, 3, 4, 5, 6, 7 oder 8 C-Atomen sind: Methyl, Ethyl, Propyl, Butyl, Pentyl, Hexyl, Heptyl, Octyl, Isopropyl, Isobutyl, Isopentyl, Neopentyl, Isohexyl, 3-Methylpentyl, sec-Butyl, tert-Butyl, tert-Pentyl.

Cycloalkylreste sind insbesondere Cyclopropyl, Cyclobutyl, Cyclopentyl, Cyclohexyl, Cycloheptyl, die aber auch beispiesweise durch Alkyl mit 1, 2, 3 oder 4 C-Atomen substituiert sein können. Als Beispiel für substituierte Cycloalkylreste seien 4-Methylcyclohexyl und 2,3-Dimethylcyclopentyl genannt.

Unter Heteroaryl mit 1,2,3,4,5,6,7,8 oder 9 C-Atomen werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Gegebenenfalls vorkommende Stereozentren können sowohl (R)- als auch (S)-konfiguriert sein.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Erfindung betrifft auch ein Verfahren zur Herstellung der neuen Verbindungen der Formel (I), sowie deren physiologisch verträglichen Salze, das dadurch gekennzeichnet ist, daß man Verbindungen der Formel (II) 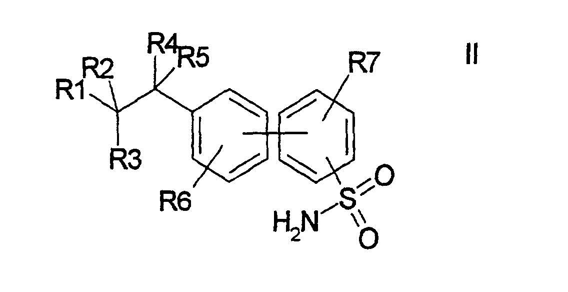 worin die Reste wie oben definiert sind mit Bromcyan umsetzt. Die Reaktion erfolgt zweckmäßigerweise in einem dipolar aprotischen Lösungsmittel, das gegen Bromcyan stabil ist, zum Beispiel Acetonitril, DMA, TMU oder NMP mit einer starken Hilfsbase, die wenig nucleophil ist wie zum Beispiel K₂CO₃ oder Cs₂CO₃. Als Reaktionstemperatur kommt eine Temperatur zwischen 0 °C und dem Siedepunkt des verwendeten Lösungsmittels in Frage, bevorzugt ist eine Temperatur zwischen 60 °C und 120 °C.

Verbindungen der Formel II können durch Wittig-Reaktion einer Verbindung der Formel III, 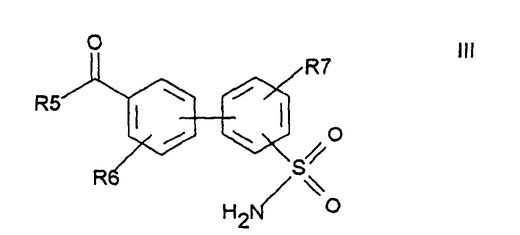 worin R(5), R(6) und R(7) wie oben definiert sind und der beispielsweise wie in Liebigs Ann. 1995 1253 beschrieben, hergestellt werden kann, mit einem Phosphoran, das die Reste R(1) und/oder R(2) und/oder R(3) enthält. Solche Wittig-Reaktionen sind dem Fachmann bekannt und zum Beispiel in Org. Synth. 1960, 40, 66; J. Org. Chem. 1963, 28, 1128 sowie Org. Synth. Coll. Vol. 5 1973, 751 beschrieben.

Die erfindungsgemäßen Verbindungen der Formel I eignen sich als Inhibitoren des Natrium-abhängigen Bicarbonat/Chlorid-Austauschers (NCBE) bzw. des NatriumlBicarbonat-Symporters.

In EP-A 855 392 sind Imidazol-Derivate mit einer Biphenylsulfonylcyanamid-Seitenkette als NCBE-Inhibitoren vorgeschlagen worden.

Des weiteren betrifft die Erfindung die Verwendung einer Verbindung der Formel I zur Herstellung eines Medikaments zur Behandlung oder Prophylaxe von durch ischämische Zustände hervorgerufenen Krankheiten;
sowie die Verwendung einer Verbindung der Formel I zur Herstellung eines Medikaments zur Behandlung oder Prophylaxe des Herzinfarkts;
sowie die Verwendung einer Verbindung der Formel I zur Herstellung eines Medikaments zur Behandlung oder Prophylaxe der Angina Pectoris;
sowie die Verwendung einer Verbindung der Formel I zur Herstellung eines Medikaments zur Behandlung oder Prophylaxe von ischämischen Zuständen des Herzens;
sowie die Verwendung einer Verbindung der Formel I zur Herstellung eines Medikaments zur Behandlung oder Prophylaxe von ischämischen Zuständen des peripheren und zentralen Nervensystems und des Schlaganfalls;
sowie die Verwendung einer Verbindung der Formel I zur Herstellung eines Medikaments zur Behandlung oder Prophylaxe von ischämischen Zuständen peripherer Organe und Gliedmaßen;
sowie die Verwendung einer Verbindung der Formel I zur Herstellung eines Medikaments zur Behandlung von Schockzuständen;
sowie die Verwendung einer Verbindung der Formel I zur Herstellung eines Medikaments zum Einsatz bei chirurgischen Operationen und Organtransplantationen;
sowie die Verwendung einer Verbindung der Formel I zur Herstellung eines Medikaments zur Konservierung und Lagerung von Transplantaten für chirurgische Maßnahmen;
sowie die Verwendung einer Verbindung der Formel I zur Herstellung eines Medikaments zur Behandlung von Krankheiten, bei denen die Zellproliferation eine primäre oder sekundäre Ursache darstellt; und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie;
sowie die Verwendung einer Verbindung der Formel I zur Herstellung eines Medikaments zur Behandlung von gestörtem Atemantrieb;
sowie ein Arzneimittel, gekennzeichnet durch einen wirksamen Gehalt einer Verbindung der Formel I.

Die erfindungsgemäßen Verbindungen der Formel (I) zeigen sehr gute antiarrhythmische Eigenschaften, wie sie beispielsweise für die Behandlung von Krankheiten wichtig sind, welche bei Sauerstoffmangelerscheinungen auftreten. Die Verbindungen der Formel (I) sind infolge ihrer pharmakologischen Eigenschaften als antiarrhythmische Arzneimittel mit cardioprotektiver Komponente zur Infarktprophylaxe und der Infarktbehandlung sowie zur Behandlung der Angina Pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern.
Wegen ihrer schützenden Wirkungen gegen pathologische hypoxische und ischämische Situationen können die erfindungsgemäßen Verbindungen der Formel (I) infolge Inhibition des zellulären Na⁺-abhängigen Cl⁻/HCO₃⁻- Austauschmechanismus (= NCBE-Inhibitoren) bzw. des Natrium/Bicarbonat-Symporters als Arzneimittel zur Behandlung aller akuten oder chronischen durch Ischämie ausgelösten Schäden oder dadurch primär oder sekundär induzierten Krankheiten verwendet werden. Sie schützen akut oder chronisch sauerstoffmangelversorgte Organe durch Verringerung oder Verhinderung ischämisch induzierter Schäden und eignen sich somit als Arzneimittel beispielsweise bei Thrombosen, Gefäßspasmen, Atherosklerose oder bei operativen Eingriffen (z.B. bei OrganTransplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder chronischem oder akutem Nierenversagen.

Die Verbindungen der Formel (I) sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel (I) ebenfalls zur Behandlung von Formen des Schocks, wie beispielsweise des allergischen, cardiogenen, hypovolämischen und des bakteriellen Schocks.

Darüberhinaus zeichnen sich die erfindungsgemäßen Verbindungen der Formel (I) durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel (I) als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Es wurde gefunden, daß Inhibitoren des Na⁺-abhängigen Cl⁻/HCO₃⁻-Austauschers (NCBE-Inhibitoren) bzw. des Natrium/Bicarbonat-Symporters die Atmung durch eine Zunahme der Chemosensibiliät der Atmungs-Chemorezeptoren stimulieren können. Diese Chemorezeptoren sind in beträchtlichem Umfang für die Aufrechterhaltung einer geordneten Atemtätigkeit verantwortlich. Sie werden durch Hypoxie, pH-Abfall und Anstieg von CO₂ (Hyperkapnie) im Körper aktiviert und führen zu einer Anpassung des Atemminutenvolumens. Im Schlaf ist die Atmung besonders störanfällig und in hohem Maße abhängig von der Aktivität der Chemorezeptoren. Eine Verbesserung des Atemantriebes durch Stimulation der Chemorezeptoren mit Substanzen, die den Na⁺-abhängigen Cl⁻/HCO₃⁻ -Austausch hemmen, führt zu einer Verbesserung der Atmung bei folgenden klinischen Zuständen und Krankheiten: Gestörter zentraler Atemantrieb (z.B. zentrale Schlafapnoen, plötzlicher Kindstod, postoperative Hypoxie), muskulär-bedingte Atemstörungen, Atemstörungen nach Langzeitbeatmung, Atemstörungen bei Adapation im Hochgebirge, obstruktive und gemischte Form der Schlafapnoen, akute und chronische Lungenkrankheiten mit Hypoxie und Hyperkapnie.

Die erfindungsgemäßen Verbindungen der Formel I und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und die Herstellung von Medikamenten dafür. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen pharmazeutisch einwandfreien Träger- und Hilfsstoffen enthalten. Die pharmazeutischen Zubereitungen enthalten normalerweise 0,1 bis 99 Gewichtsprozent, bevorzugt 0,5 bis 95 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die eine Verbindung der Formel (I) und/oder ihre physiologisch verträglichen Salze enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken-als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs einer Verbindung der Formel (I) und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Die Verbindungen der Formel I und/oder ihre physiologisch verträglichen Salze können zur Erzielung einer vorteilhaften therapeutischen Wirkung auch zusammen mit anderen pharmakologisch wirksamen Verbindungen zur Behandlung oder Prophylaxe der obengenannten Krankheitsbilder, insbesonders zur Behandlung von Herz-Kreislauf-Erkrankungen eingesetzt werden. Bevorzugt ist die Kombination mit Inhibitoren des Natrium/Wasserstoff-Austauschers (NHE) und/oder mit aktiven Substanzen aus anderen Herz-Kreislauf-Wirkstoffklassen.

Die Erfindung betrifft desweiteren ganz allgemein die Kombination von a) NCBE-Inhibitoren und/oder deren physiologisch verträglichen Salze mit NHE-Inhibitoren und/oder deren physiologisch verträglichen Salze; b) NCBE-Inhibitoren und/oder deren physiologisch verträglichen Salze mit aktiven Substanzen aus anderen Herz-Kreislauf-Wirkstoffklassen und/oder deren physiologisch verträglichen Salze sowie c) von NCBE-Inhibitoren und/oder deren physiologisch verträglichen Salze mit NHE-Inhibitoren und/oder deren physiologisch verträglichen Salze und mit aktiven Substanzen aus anderen Herz-Kreislauf-Wirkstoffklassen und/oder deren physiologisch verträglichen Salze. Bevorzugt sind solche Kombinationen, in denen NCBE-Inhibitoren der allgemeinen Formel I und/oder deren physiologisch verträglichen Salze verwendet werden.

Bei den bekannten und als NHE-Inhibitoren identifizierten Wirkstoffen handelt es sich um Guanidin-Derivate, vorzugsweise um Acylguanidine, unter anderem wie sie in Edward J. Cragoe, Jr., "DIURETICS, Chemistry, Pharmacology and Medicine", J. WILEY & Sons (1983), 303 - 341 beschrieben sind oder um die in DE19737224.4 aufgeführten NHE-Inhibitoren.
Geeignete NHE-Inhibitoren sind beispielsweise auch Benzoylguanidine, wie sie in US 5292755, US 5373024, US 5364868, US 5591754, US 5516805, US 5559153, US 5571842, US 5641792, US 5631293, EP-A 577024, EP-A 602522, EP-A 602523, EP-A 603650, EP-A 604852, EP-A 612723, EP-A 627413, EP-A 628543, EP-A 640593, EP-A 640588, EP-A702001, EP-A 713864, EP-A 723956, EP-A 754680, EP-A 765868, EP-A 774459, EP-A 794171, DE 19624178.2, DE 19713427.0 beschrieben sind; ortho-substituierte Benzoylguanidine, wie sie in EP-A 556673, EP-A 791577, EP-A 794172, DE 19624178.2 beschrieben sind; orthoamino-substituierte Benzoyl-guanidine, wie sie in EP-A 690048 beschrieben sind; Isochinoline, wie sie in EP-A 590455 beschrieben sind; Benzokondensierte 5-Ring-Heterocyclen, wie sie in EP-A 639573 beschrieben sind; Diacylsubstituierte Guanidine, wie sie in EP-A 640587 beschrieben sind; Acylguanidine, wie sie in US 5547953 beschrieben sind; Perfluoralkylgruppen tragende Phenylsubstituierte Alkylbzw. Alkenyl-carbonsäure- Guanidine, wie sie in US 5567734, EP-A 688766 beschrieben sind; Heteroaroyl-guanidine, wie sie in EP-A 676395 beschrieben sind; Bizyklische Heteroaroyl-guanidine, wie sie in EP-A 682017 beschrieben sind; Indenoylguanidine, wie sie in EP-A 738712 beschrieben sind; Benzyloxycarbonylguanidine, wie sie in EP-A 748795 beschrieben sind; Fluorphenylgruppen tragende Phenylsubstituierte Alkenylcarbonsäure-Guanidine, wie sie in EP-A 744397 beschrieben sind; substituierte Zimtsäureguanidine, wie sie in EP-A 755919 beschrieben sind; Sulfonimidamide, wie sie in EP-A 771788 beschrieben sind; Benzoldicarbonsäure-diguanidine, wie sie in EP-A 774458, EP-A 774457 beschrieben sind; Diarylcarbonsäure-diguanidine, wie sie in EP-A 787717 beschrieben sind; substituierte Thiophenylalkenylcarbonsäureguanidine, wie sie in EP-A 790245 beschrieben sind; Bis-ortho-substituierte Benzoylguanidine, wie sie in DE19621319.3beschrieben sind; Substituierte 1-oder2-Naphthylguanidine, wie sie in DE 19621482.3 und DE 19621483.1 beschrieben sind; Indanylidinacetylguanidine, wie sie in EP-A 837055 beschrieben sind; Phenylsubstituierte Alkenylcarbonsäure-guanidine, wie sie in DE 19633966.9 beschrieben sind; Aminopiperidyl-Benzoylguanidine, wie sie in EP 667341 beschrieben sind; Heterocycloxy-Benzylguanidine, wie sie in EP-A 694537 beschrieben sind; ortho-substituierte Benzoylguanidine, wie sie in EP704431 beschrieben sind; ortho-substituierte Alkyl-Benzylguanidine, wie sie in EP-A 699660 beschrieben sind; ortho-substituierte Heterocyclyl-Benzoylguanidine, wie sie in EP-A 699666 beschrieben sind; ortho-substituierte 5-Methylsulfonyl-benzoylguanidine, wie sie EP-A 708088 beschrieben sind; ortho-substituierte 5-Alkylsulfonyl-benzoylguanidine mit 4-amino Substituenten, wie sie in EP-A 723963 beschrieben sind; ortho-substituierte 5-Alkylsulfonyl-Benzoylguanidine mit 4-Mercapto Substituenten, wie sie in EP-A 743301 beschrieben sind; 4-Sulfonyl- oder 4-Sulphinyl-Benzylguanidine, wie sie in EP-A 758644 beschrieben sind; Alkenylbenzoylguanidine, wie sie in EP-A 760365 beschrieben sind; Benzoylguanidine mit annelierten, cyclischen Sulfonen, wie sie in DE 19548708 beschrieben sind; Benzoyl-, polycyclische Aroyl- und Heteroaroyl-guanidine, wie sie in WO 9426709 beschrieben sind; 3-Aryl/Heteroaryl-Benzoylguanidine, wie sie in WO 9604241 beschrieben sind; 3-Phenyl-Benzoylguanidine mit einem basischen Amid in der 5-Position, wie sie in WO 9725310 beschrieben sind; 3-Dihalothienyl- oder 3-Dihalophenyl-Benzoylguanidine mit einem basichen Substituenten in der 5-Position, wie sie in WO 9727183 beschrieben sind; 3-Methylsulfonylbenzoylguanidinen mit bestimmten Aminosubstituenten in der 4-Position, wie sie in WO 9512584 beschrieben sind; Amilorid-Derivate, wie sie in WO 9512592 beschrieben sind; 3-Methylsulfonylbenzoylguanidine mit bestimmten Aminosubstituenten in der 4-Position, wie sie in WO 9726253 beschrieben sind; Indoloylguanidine, wie sie in EP-A 622356 und EP-A 708091 beschrieben sind; Indoloylguanidine mit einem annelierten zusätzlichen Ringsystem, wie sie in EP 787728 beschrieben sind; Methylguanidin-Derivate, wie sie in WO 9504052 beschrieben sind; 1,4-Benzoxazinoylguanidine, wie sie in EP-A 719766 beschrieben sind; 5-Brom-2-Naphthoylguanidine, wie sie in JP 8225513 beschrieben sind; Quinolin-4-Carbonylguanidine mit einem Phenylrest in 2-Position, wie sie in EP-A 726254 beschrieben sind; Cinnamoylguanidine, wie sie in JP 09059245 beschrieben sind; Propenoylguanidine mit einem Naphthalin-Substituenten, wie sie in JP 9067332 beschrieben sind; Propenoylguanidine mit Indolsubstituenten, wie sie in JP 9067340 beschrieben sind; oder Heteroaryl-substituierte Acroylguanidine, wie sie in WO 9711055 beschrieben sind, sowie deren physiologisch verträglichen Salze.

Bevorzugte NHE-Inhibitoren sind die in den genannten Publikationen als bevorzugt hervorgehobenen Verbindungen. Ganz besonders bevorzugt sind die Verbindungen Cariporid (HOE642), HOE 694, EMD 96785, FR 168888, FR 183998, SM-20550, KBR-9032, sowie deren physiologisch verträglichen Salze. Am meisten bevorzugt ist Cariporid oder ein anderes physiologisch verträgliches Salz des N-(4-Isopropyl-3-methansulfonyl-benzoyl)-guanidin.

Beispiele für herzkreislaufaktive Wirkstoffklassen, die sich therapeutisch vorteilhaft mit NCBE-Inhibitoren kombinieren lassen oder zusätzlich mit Kombinationen von NCBE-Inhibitoren und NHE-Inhibitoren kombiniert werden können, sind Beta-Rezeptoren-Blocker, Calcium-Antagonisten, Angiotensin-Conversions-Enzym-Hemmer, Angiotensin-Rezeptorblocker, Schleifendiuretika, Thiaziddiuretika, kaliumsparende Diuretika, Aldosteronantagonisten, wie sie beispielsweise in der Blutdrucksenkung eingesetzt werden, sowie Herzglykoside oder andere kontraktionskraftverstärkende Mittel in der Behandlung der Herzinsuffizienz und des Congestive Heart Failures, sowie Antiarrhythmika der Klassen I - IV, Nitrate, K_{ATP}-Öffner, K_{ATP}-Blocker, Hemmer des Veratridin-aktivierbaren Natriumkanals usw. So sind zum Beispiel geeignet: die Betablocker Propanolol, Atenolol, Metoprolol; die Calciumantagonisten Diltiazem-Hydrochlorid, Verapamil-Hydrochlorid, Nifedipin; die ACE-Hemmer Captopril, Enalapril, Ramipril; Trandolapril, Quinapril, Spirapril, bevorzugt Ramipril oder Trandolapril; die Angiotensin II-Rezeptorantagonisten Losartan, Valsartan, Telmisartan, Eprosartan, Tasosartan, Candesartan, Irbesartan; die Schleifendiruetika Furosemid, Piretanid, Torasemid; die Thiazid-Diuretika Hydrochlorothiazid, Metolazon, Indapamid; die kaliumsparenden Diuretika Amilorid, Triamteren, Spironolacton; die Herzglykoside Digoxin, Digitoxin, Strophanthin; die Antiarrhythmika Amiodaron, Sotalol, Bretylium, Flecainid; das Nitrat Glyceroltrinitrat; die K⁺(ATP)-Öffner Cromakalim, Lemakalim, Nocorandil, Pinacidil, Minoxidil; die Hemmer des veratridin-aktivierbaren Na⁺-Kanals.

Ein Beispiel eines solchen besonders vorteilhaften Kombinationspartners mit NCBE-Inhibitoren sind Blocker des nicht-inaktivierenden-Natriumkanals (Veratridin-aktivierbarer Natriumkanal). Die Kombinationen eines NCBE-Inhibitors mit einem Blocker des nicht-inaktivierenden-Natriumkanals (Veratridin-aktivierbarer Natriumkanal) eignen sich zur Infarkt- und Re-infarktprophylaxe und der Infarktbehandlung sowie zur Behandlung der Angina Pectoris und der Inhibierung ischämisch induzierter Herzarrhythmien, der Tachykardie und der Entstehung und Aufrechterhaltung des Kammerflimmerns, wobei die Kombinationen eines NCBE-Inhibitors mit einem Blocker des nicht-inaktivierenden-Natriumkanals auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen eines NCBE-Inhibitors mit einem Blocker des nicht-inaktivierenden-Natriumkanals infolge verstärkter Inhibierung des Na+ Einstroms in die Zelle als Arzneimittel zur Behandlung aller akuten oder chronischen, durch Ischämie ausgelösten Schäden oder dadurch primär oder sekundäre induzierter Krankheiten verwendet werden. Dies betrifft ihre Verwendung als Arzneimittel für operative Eingriffe, z. B. bei Organtransplantationen, wobei die Kombinationen eines NCBE-Inhibitors mit einem Blocker des nicht-inaktivierenden-Natriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NCBE-Inhibitors mit einem Blocker des nicht-inaktivierenden-Natriumkanals sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind die Kombinationen eines NCBE-Inhibitors mit einem Blocker des nicht-inaktivierenden-Natriumkanals auch als Arzneimittel zur Behandlung von Ischämien des Nervensystems, insbesondere des Zentralnervensystems geeignet, wobei sie zur Behandlung des Schlaganfalls oder des Himödems geeignet sind. Darüber hinaus eignen sich die erfindungsgemäßen Kombinationen eines NCBE-Inhibitors mit einem Blocker des nicht-inaktivierenden-Natriumkanals ebenfalls zur Behandlung von Formen des Schocks, wie beispielsweise des allergischen, cardiogenen, hypovolämischen und des bakteriellen Schocks.

Neben der Anwendung als fixe Kombination betrifft die Erfindung auch die gleichzeitige, getrennte oder zeitlich abgestufte Anwendung von NCBE-Inhibitoren mit NHE-Inhibitoren und/oder einer zusätzlichen aktiven Substanz aus einer anderen Herz-Kreislauf-Wirkstoffklasse zur Behandlung der obengenannten Krankheiten.

Die Erfindung betrifft desweiteren eine pharmazeutische Zubereitung, enthaltend a) einen NCBE-Inhibitor und einen NHE-Inhibitor und/oder deren physiologisch verträglichen Salze; oder b) einen NCBE-Inhibitor und zusätzlich eine aktive Substanz aus einer anderen Herz-Kreislauf-Wirkstoffklasse und/oder deren physiologisch verträglichen Salze; oder c) einen NCBE-Inhibitor, einen NHE-Inhibitor und zusätzlich eine aktive Substanz aus einer anderen Herz-Kreislauf-Wirkstoffklasse, und/oder deren physiologisch verträglichen Salze.

Bevorzugt sind pharmazeutische Zubereitungen, die eine Verbindung der Formel I und/oder deren physiologisch verträgliches Salz als NCBE-Inhibitor enthalten.

Durch die kombinierte Anwendung kann der Effekt des einen Kombinationspartners durch den jeweiligen anderen Partner potenziert werden, d.h., die Wirkung und/oder Wirkdauer einer erfindungsgemäßen Kombination oder Zubereitung ist stärker bzw. anhaltender als die Wirkung und/oder Wirkdauer der jeweiligen Einzelkomponenten (synergistischer Effekt). Dies führt bei einer kombinierten Anwendung zu einer Verringerung der Dosis der jeweiligen Kombinationspartner, verglichen mit der Einzelanwendung. Die erfindungsgemäßen Kombinationen und Zubereitungen besitzen demnach den Vorteil, daß die zu applizierenden Wirkstoffmengen signifikant reduziert und unerwünschte Nebenwirkungen eliminiert bzw. stark redzuziert werden können.

Die Erfindung betrifft ferner eine Handelspackung, enthaltend als pharmazeutischen Wirkstoff a) einen NCBE-Inhibitor und einen NHE-Inhibitor und/oder deren physiologisch verträglichen Salze; oder b) einen NCBE-Inhibitor und zusätzlich eine aktive Substanz aus einer anderen Herz-Kreislauf-Wirkstoffklasse und/oder deren physiologisch verträglichen Salze; oder c) einen NCBE-Inhibitor, einen NHE-Inhibitor und zusätzlich eine aktive Substanz aus einer anderen Herz-Kreislauf-Wirkstoffklasse und/oder deren physiologisch verträglichen Salze jeweils zusammen mit Instruktionen für die Verwendung dieser Wirkstoffe in Kombination zur gleichzeitigen, getrennten oder zeitlich abgestuften Anwendung in der Behandlung oder Prophylaxe der obengenannten Krankheitsbilder, insbesondere zur Behandlung von Herz-Kreislauf-Erkrankungen. Bevorzugt sind Handelspackungen, die Verbindungen der Formel I als NCBE-Inhibitoren enthalten.

Die erfindungsgemäßen pharmazeutischen Zubereitungen können beispielsweise hergestellt werden, indem man entweder die Einzelkomponenten als Pulver intensiv mischt, oder indem man die Einzelkomponenten in den geeigneten Lösungsmittel wie beispielsweise einem niederen Alkohol auflöst und das Lösungsmittel anschließend entfernt.

Das Gewichtsverhältnis vom NCBE-Inhibitor zum NHE-Inhibitor oder der herzkreislaufaktiven Substanz in den erfindungsgemäßen Kombinationen und Zubereitungen beträgt zweckmässigerweise 1:0,01 bis 1:100 , vorzugsweise 1:0,1 bis 1:10.

Die erfindungsgemäßen Kombinationen und Zubereitungen enthalten insgesamt vorzugsweise 0,5-99,5 Gew.-%, insbesondere 4-99 Gew.-% dieser Wirkstoffe.

Bei der erfindungsgemäßen Anwendung bei Säugern, vorzugsweise beim Menschen bewegen sich beispielsweise die Dosen der verschiedenen Wirkstoffkomponenten im Berreich von 0,001 bis 100 mg/kg/Tag.

### Liste der Abkürzungen:

- BCECF: 2',7' - Bis (2-carboxyethyl)-5,6-carboxyfluorescein
- Bn: Benzyl
- CH₂Cl₂: Dichlormethan
- DCI: Desorption-Chemical Ionisation
- DIP: Diisopropylether
- DMA: Dimethylacetamid
- DME: Dimethoxyethan
- DMF: N, N-Dimethylformamid
- EE: Ethylacetat (EtOAc)
- El: electron impact
- eq: Äquivalent
- ES: Elektrospray-lonisation
- ESneg: Elektrospray, negative Ionisation
- Et: Ethyl
- EtOH: Ethanol
- FAB: Fast Atom Bombardment
- HEP: n-Heptan
- HOAc: Essigsäure
- KOtBu: Kalium-*t*-butylat
- Me: Methyl
- MeOH: Methanol
- mp: Schmelzpunkt
- MTB: Methyltertiärbutylether
- NCBE: Natriumabhängiger Chlorid/Bicarbonat-Austauscher
- NHE: Natrium/Wasserstoff-Austauscher
- NMP: N-Methylpyrrolidon
- RT: Raumtemperatur
- THF: Tetrahydrofuran
- TMU: N, N, N', N'-Tetramethylharnstoff
- Tol: Toluol
- ZNS: Zentralnervensystem

### Allgemeine Vorschrift zur Herstellung von Sulfonylcyanamiden aus Sulfonamiden

Das Sulfonamid-Ausgangsmaterial wird in 10 ml/mmol wasserfreiem Acetonitril gelöst, 3 Mol-Äquivalente K₂CO₃ sowie ein Mol-Äquivalent einer 5 N Lösung von BrCN in Acetonitril zugetropft und bis zum vollständigen Umsatz unter Rückfluß erhitzt (typische Reaktionszeit 10 Minuten bis 6 Stunden). Das Reaktionsgemisch wird anschließend nach Abkühlung auf RT ohne weitere Aufarbeitung an Kieselgel chromatographiert.

### Beispiel 1 4'-Cyclohexylidenmethyl-biphenyl-2-sulfonylcyanamid

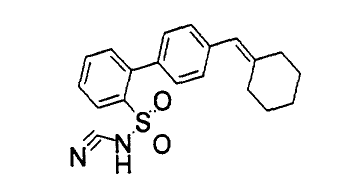

### a) 4'-Formyl-biphenyl-2-sulfonamid

5.0 g 4'-Formyl-biphenyl-2-sulfonsäure-dimethylaminomethylenamid (Liebigs Ann. 1995, 1253) wurden in 50 ml EtOH gelöst, mit 50 ml einer gesättigten wäßrigen HCI-Lösung versetzt und 2 h unter Rückfluß gekocht. Anschließend wurde auf RT abgekühlt, 500 ml Wasser wurden hinzugegeben, 2 h gerührt und das Produkt abgesaugt. Aus MTB wurde umkristallisiert und man erhielt 2.8 g weißer Kristalle, mp 165 °C (unter Zersetzung).
R_{f} (MTB) = 0.57 MS (DCI): 262 (M+1)⁺

### b) 4'-Cyclohexylidenmethyl-biphenyl-2-sulfonamid

5.5 g Cyclohexyl-triphenylphosphonium-bromid und 2.6 g KOtBu wurden in 200 ml wasserfreiem THF 4 h bei RT gerührt. 3.0 g 4'-Formyl-biphenyl-2-sulfonamid wurden zugegeben und 18 h bei RT gerührt. Anschließend wurde mit 200 ml EE verdünnt, mit wäßriger HCI-Lösung auf pH = 6-7 gestellt und 2 mal mit je 100 ml einer gesättigten wäßrigen NaCl-Lösung gewaschen. Über Na₂SO₄ wurde getrocknet und das Solvens im Vakuum entfernt. Chromatographie an Kieselgel mit DIP lieferte 1,5 g eines farblosen Öls.
R_{f} (DIP) = 0.36 MS (DCI): 328 (M+1)⁺

### c) 4'-Cyclohexylidenmethyl-biphenyl-2-sulfonylcyanamid

267 mg 4'-Cyclohexylidenmethyl-biphenyl-2-sulfonamid wurden zu der Titelverbindung umgesetzt. Man erhält 80 mg eines blaßgelben Öls.
R_{f} (EE/MeOH 10:1) = 0.17

### Beispiel 2 4'-Cyclohexymethyl-biphenyl-2-sulfonylcyanamid

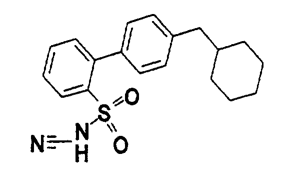

### a) 4'-Cyclohexylmethyl-biphenyl-2-sulfonamid

1,45 g 4'-Cyclohexylidenmethyl-biphenyl-2-sulfonamid wurden in 50 ml MeOH gelöst und mit 200 mg Pd/C (10%) versetzt. 20 h lang wurde unter H₂ bei RT unter Normaldruck hydriert, anschließend wurde der Katalysator abgesaugt und das Solvens im Vakuum entfernt. Man erhielt 1,4 g eines farblosen Öls.
R_{f} (DIP) = 0.45 MS (DCI): 330 (M+1)⁺

### b) 4'-Cyclohexylmethyl-biphenyl-2-sulfonylcyanamid

1,4 g 4'-Cyclohexylmethyl-biphenyl-2-sulfonamid wurden nach der allgemeinen Vorschrift zur Herstellung von Sulfonylcyanamiden aus Sulfonamiden 2 Stunden umgesetzt und man erhielt 1,2 g eines farblosen Schaums.
R_{f} (EE/MeOH 10:1) = 0,31 IR (-C≡N): 2183,1 cm⁻¹

Die Titelverbindungen der Beispiele 3 und 4 wurden analog Beispiel 1 und 2 synthetisiert:

### Beispiel 3 4'-Cyclopentylidenmethyl-biphenyl-2-sulfonylcyanamid

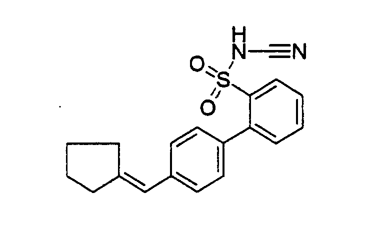

1,5 Stunden umgesetzt; farblose Kristalle, mp. 115-120 °C unter Zersetzung R_{f} (EE/MeOH 10:1) = 0.18 IR (-C≡N): 2180,3 cm⁻¹ MS (ESneg) : 337 (M-1)⁻

### Beispiel 4 4'-Cycloheptylidenmethyl-biphenyl-2-sulfonylcyanamid

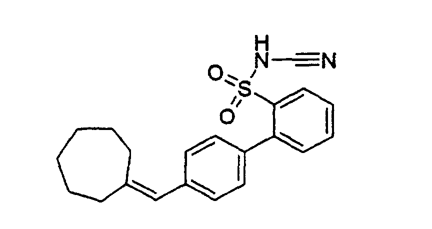

1,5 Stunden umgesetzt; R_{f} (EE/MeOH 10:1) = 0,17 IR (-C≡N): 2180,8 cm⁻¹ MS (ESneg) : 365 (M-1)⁻
mp. Kaliumsalz 168-171 °C unter Zersetzung.

### Beispiel 5 cis-4'-Styryl-biphenyl-2-sulfonylcyanamid

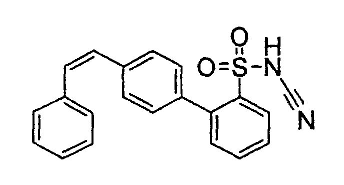

### a) 4'-Styryl-biphenyl-2-sulfonamid

9,0 g Benzyl-triphenylphosphonium-chlorid und 2.6 g KOtBu wurden in 80 ml wasserfreiem THF 4 h bei RT gerührt. Anschließend wurden 3,0 g 4'-Formylbiphenyl-2-sulfonamid zugegeben und 20 h bei RT gerührt. 200 ml einer gesättigten wäßrigen NaHCO₃-Lösung wurden zugegeben und 2 mal mit je 200 ml EE extrahiert. Über Na₂SO₄ wurde getrocknet und das Solvens im Vakuum entfernt.

Chromatographie an Kieselgel mit DIP/HEP 1:2 lieferte 600 mg cis-4'-Styrylbiphenyl-2-sulfonamid, R_{f} (DIP/HEP 1:2) = 0,25 MS (DCI): 336 (M+1)⁺ und 1,2 g trans-4'-Styryl-biphenyl-2-sulfonamid, R_{f} (DIP/HEP 1:2) = 0.20 MS (DCI): 336 (M+1)⁺

### b) cis-4'-Styryl-biphenyl-2-sulfonylcyanamid

300 mg cis-4'-Styryl-biphenyl-2-sulfonamid wurden nach der allgemeinen Vorschrift zur Herstellung von Sulfonylcyanamiden aus Sulfonamiden 2 Stunden umgesetzt und man erhielt 249 mg eines farblosen Schaums.
R_{f} (EE/MeOH 10:1) = 0,26 IR (-C≡N): 2182.3 cm⁻¹ MS (ESneg): 359 (M-1)⁻ mp. Kaliumsalz 190 °C unter Zersetzung.

### Beispiel 6 trans-4'-Styryl-biphenyl-2-sulfonylcyanamid

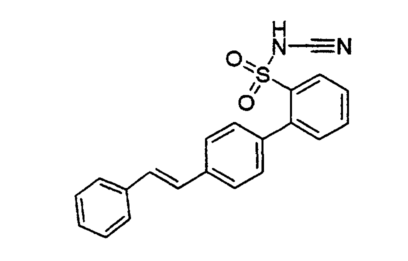

300 mg trans-4'-Styryl-biphenyl-2-sulfonamid wurden nach der allgemeinen Vorschrift zur Herstellung von Sulfonylcyanamiden aus Sulfonamiden 2 Stunden umgesetzt und man erhielt 290 mg eines farblosen Schaums.

R_{f} (EE/MeOH 10:1) = 0,21 IR (-C≡N): 2180,8 cm⁻¹ MS (ESneg): 359 (M-1)⁻ mp. Kaliumsalz 170 °C unter Zersetzung.

### Beispiel 7 4'-Phenethyl-biphenyl-2-sulfonylcyanamide

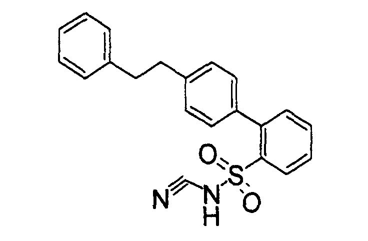

### a) 4'-Phenethyl-biphenyl-2-sulfonamid

400 mg 4'-Styryl-biphenyl-2-sulfonamid wurden in 10 ml MeOH gelöst und 80 mg Pd/C (10%) zugegeben.
20 h lang wurde unter H₂ bei RT unter Normaldruck hydriert, anschließend wurde der Katalysator abgesaugt und das Solvens im Vakuum entfernt. Man erhielt 350 mg eines farblosen Öls.
R_{f} (DIP) = 0,46 MS (DCI): 338 (M+1)⁺

### b) 4'-Phenethyl-biphenyl-2-sulfonylcyanamid

340 mg 4'-Phenethyl-biphenyl-2-sulfonamid wurden nach der allgemeinen Vorschrift zur Herstellung von Sulfonylcyanamiden aus Sulfonamiden 1 Stunde umgesetzt und man erhielt 360 mg eines farblosen Schaums.
R_{f} (EE/MeOH 10:1) = 0,28 IR (-C≡N): 2178,3 cm⁻¹

Die Titelverbindung des Beispieles 8 wurde analog Beispiel 5 synthetisiert:

### Beispiel 8 cis-4'-(2-Cyclohexyl-vinyl)-biphenyl-2-sulfonylcyanamid

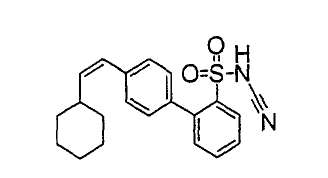

### a) cis-4'-(2-Cyclohexyl-vinyl)-biphenyl-2-sulfonamid

R_{f} (DIP) = 0,45 MS (ES): 342 (M+1)⁺

### b) cis-4'-(2-Cyclohexyl-vinyl)-biphenyl-2-sulfonylcyanamid

Umsetzungszeit: 2 Stunden; R_{f} (EE/MeOH 10:1) = 0,17 IR (-C≡N): 2176,9 cm⁻¹ MS (ESneg) : 365 (M-1)⁻ mp. Kaliumsalz 189-193 °C unter Zersetzung.

### Beispiel 9 trans-4'-(2-Cyclohexyl-vinyl)-biphenyl-2-sulfonylcyanamid

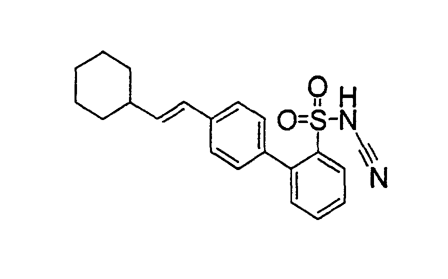

### a) trans-4'-(2-Cyclohexyl-vinyl)-biphenyl-2-sulfonamid

1,2 g cis-4'-(2-Cyclohexyl-vinyl)-biphenyl-2-sulfonamid und 894 mg lod wurden in 100 ml wasserfreiem CH₂Cl₂ gelöst und 5 Tage bei RT stehen gelassen. Das Reaktionsgemisch wurde mit 200 ml CH₂Cl₂ verdünnt und 2 mal mit je 100 ml einer gesättigten wäßrigen Na₂SO₃-Lösung gewaschen. Über Na₂SO₄ wurde getrocknet und das Solvens im Vakuum entfernt. Chromatographie an Kieselgel mit DIP lieferte 260 mg eines farblosen Öls.
R_{f} (DIP) = 0,45 MS (ES) : 342 (M+1)⁺

### b) trans-4'-(2-Cyclohexyl-vinyl)-biphenyl-2-sulfonylcyanamid

260 mg trans-4'-(2-Cyclohexyl-vinyl)-biphenyl-2-sulfonamid wurden nach der allgemeinen Vorschrift zur Herstellung von Sulfonylcyanamiden aus Sulfonamiden 2 Stunden umgesetzt und man erhielt 90 mg eines farblosen Öls.
R_{f} (EE/MeOH 10:1) = 0,09 IR (-C≡N): 2181,9 cm⁻¹ MS (ESneg): 365 (M-1)⁻

### Beispiel 10 4'-(2-Cyclohexyl-ethyl)-biphenyl-2-sulfonylcyanamid

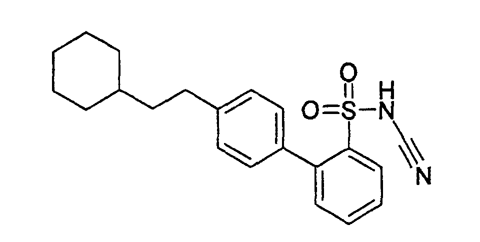

### a) 4'-(2-Cyclohexyl-ethyl)-biphenyl-2-sulfonamid

800 mg cis-4'-(2-Cyclohexyl-vinyl)-biphenyl-2-sulfonamid wurden in 10 ml MeOH gelöst und 80 mg Pd/C (10%) zugegeben. 5 h lang wurde unter H₂ bei RT unter Normaldruck hydriert, anschließend wurde der Katalysator abgesaugt und das Solvens im Vakuum entfernt. Man erhielt 760 mg eines farblosen Öls.
R_{f} (DIP) = 0,38 MS (DCI): 344 (M+1)⁺

### b) 4'-(2-Cyclohexyl-ethyl)-biphenyl-2-sulfonylcyanamid

250 mg 4'-(2-Cyclohexyl-ethyl)-biphenyl-2-sulfonamid wurden nach der allgemeinen Vorschrift zur Herstellung von Sulfonylcyanamiden aus Sulfonamiden 3 Stunden umgesetzt und man erhielt 90 mg eines farblosen Öls.
R_{f} (EE/MeOH 10:1) = 0,25 IR (-C≡N): 2178.5 cm⁻¹ MS (ESneg): 367 (M-1)⁻ mp Kaliumsalz : 193 °C unter Zersetzung

### Pharmakologische Daten:

### Inhibition des Na⁺- abhängigen Cl⁻/HCO₃⁻-Austauschers (NCBE) in humanen Endothelzellen

Humane Endothelzellen (ECV-304) wurden aus Kulturflaschen mit Hilfe von Trypsin/EDTA-Puffer (0,05/0,02% in Phoshatpuffer) abgelöst und nach Zentrifugation (100g, 5 min) in einer gepufferten Salzlösung (mmol/l: 115 NaCl, 20 NH₄Cl, 5 KCl, 1 CaCl₂, 1 MgSO₄, 20 N-(2-Hydroxyethyl)piperazin-N'-2-ethansulfonsäure (HEPES), 5 Glucose und 1 g/l Rinderserumalbumin; pH 7,4) aufgenommen. Diese Zellsuspension wurde mit 5 µM BCECF-acetoxymethylester für 20 min bei 37°C inkubiert. Anschließend wurden die Zellen gewaschen und in einer Natrium- und Bicarbonat-freien Pufferlösung (mmol/l: 5 HEPES, 133,8 Cholinchlorid, 4,7 KCI, 1,25 MgCl₂, 0,97 K₂HPO₄, 0,23 KH₂PO₄, 5 Glucose; pH 7,4) resuspendiert.
Für die nachfolgende Fluoreszenzmessung im FLIPR (Fluorescent Imaging Plate Reader) wurden pro well einer 96-well-Mikrotiterplatte 100 µl dieser Zellsuspension mit jeweils 20000 Zellen einpipettiert und diese Mikrotiterplatte zentrifugiert (100g, 5 min).
Im FLIPR wurden nun aus einer weiteren vorbereiteten Mikrotiterplatte jeweils 100 µl Pufferlösung entnommen und in jedes der 96 wells der Meßplatte einpipettiert. Dabei wurde für eine 100% Kontrolle, d.h. eine Erholung des intrazellulären pH (pHᵢ) über den NCBE, eine Bicarbonat-und Natrium-haltige Pufferlösung (mmol/l: 5 HEPES, 93,8 NaCI, 40 NaHCO₃, 4,7 KCI, 1,25 CaCl₂, 1,25 MgCl₂, 0,97 Na₂HPO₄, 0,23 NaH₂PO₄, 5 Glucose; pH 7,4) verwendet, die 50 µM HOE 642 enthielt. Für eine 0% Kontrolle, d.h. keinerlei pHᵢ-Erholung, wurde eine Bicarbonat-freie, Natrium-haltige Pufferlösung (mmol/l: 5 HEPES, 133,8 NaCI, 4,7 KCI, 1,25 CaCl₂, 1,25 MgCl₂, 0,97 Na₂HPO₄, 0,23 NaH₂PO₄, 5 Glucose; pH 7,4) eingesetzt, welcher ebenfalls 50 µM HOE 642 zugefügt waren. Die erfindungsgemäßen Verbindungen der Formel (I) wurden in verschiedenen Konzentrationen der Natrium-und Bicarbonat-haltigen Lösung zugesetzt.
Nach Zugabe der Pufferlösungen zu den in der Meßplatte befindlichen farbstoffbeladenen, angesäuerten Zellen wurde der Anstieg der Fluoreszenzintensität, welcher einem Anstieg des pHᵢ entsprach, in jedem well der Mikrotiterplatte bestimmt. Die Kinetiken wurden dabei über einen Zeitraum von 2 Minuten bei 35°C aufgenommen.
Die Zunahme der Fluoreszenzintensitäten für unterschiedliche Konzentrationen der erfindungsgemäßen Verbindungen wurden auf die beiden Kontrollen bezogen und daraus die Hemmwirkung der Substanzen ermittelt.

### Ergebnisse

### Restaktivität des NCBE bei einer Inhibitorkonzentration von 10 µM

| Beispiel | Restaktivität in % |
|---|---|
| 1 | 68.4 |
| 2 | 51.6 |
| 3 | 29.4 |
| 4 | 30.5 |
| 5 | 21.2 |
| 6 | 19.3 |
| 7 | 64.2 |
| 8 | 13.4 |
| 9 | 12.8 |
| 10 | 24.3 |

## Patentansprüche

1. Verbindungen der Formel (I), 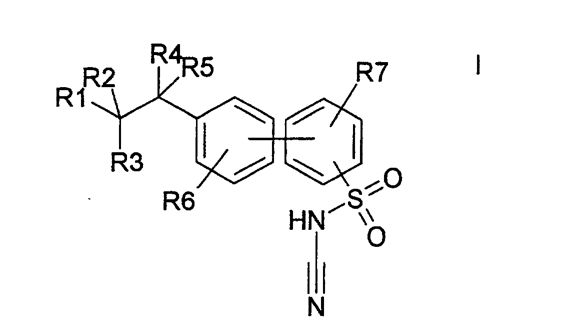 in welcher die Symbole folgende Bedeutung haben:
R(1) Wasserstoff, Alkyl mit 1,2,3,4,5,6,7 oder 8 C-Atomen, 1-Naphthyl, 2-Naphthyl, -CₐH₂ₐ-Cycloalkyl mit 3,4,5,6 oder 7 C-Atomen oder -CₐH₂ₐ-Phenyl, wobei der Phenylteil unsubstituiert oder substituiert ist mit 1-3 Substituenten aus der Gruppe Alkyl mit 1,2,3,4,5,6,7 oder 8 C-Atomen, F, Cl, Br, I, CF₃, SOₙR(11), OR(17), NR(8)R(9), -C≡N, -NO₂ oder CO-R(22);
R(11) Alkyl mit 1,2,3 oder 4 C-Atomen oder NR(20)R(21);
R(20) und R(21) unabhängig voneinander Wasserstoff oder Alkyl mit 1,2,3 oder 4 C-Atomen;
R(17) Wasserstoff oder Alkyl mit 1,2,3 oder 4 C-Atomen;
R(8) und R(9) unabhängig voneinander Wasserstoff oder Alkyl mit 1,2,3 oder 4 C-Atomen;
R(22) Wasserstoff, Alkyl mit 1,2,3,4,5,6,7 oder 8 C-Atomen oder OR(30);
R(30) Wasserstoff, Alkyl mit 1,2,3,4,5,6,7 oder 8 C-Atomen;
a Null, 1 oder 2;
n Null, 1 oder 2;
oder
R(1) und R(3) gemeinsam mit dem sie tragenden C-Atom Cycloalkyl mit 3, 4, 5, 6 oder 7 C-Atomen oder Fluorenyl;
R(2), R(3), R(4) und R(5) unabhängig voneinander Wasserstoff, F, CF₃, O-R(10), Alkyl mit 1,2,3,4,5,6,7 oder 8 C-Atomen, Cycloalkyl mit 3, 4, 5, 6 oder 7 C-Atomen, -C_{g}H_{2g}-Phenyl, wobei der Phenylteil unsubstituiert oder substituiert ist mit 1-3 Substituenten aus der Gruppe F, Cl, Br, I, CF₃, Methyl, Methoxy, Hydroxy oder NR(18)R(19);
R(18) und R(19) unabhängig voneinander Wasserstoff oder Alkyl mit 1,2,3 oder 4 C-Atomen;
g Null, 1 oder 2;
R(10) Wasserstoff, Alkyl mit 1,2,3,4,5,6,7 oder 8 C-Atomen, Phenyl, das unsubstituiert oder substituiert ist mit 1-3 Substituenten aus der Gruppe F, Cl, Br, I, CF₃, Methyl, Methoxy, Hydroxy oder NR(12)R(13);
R(12) und R(13) unabhängig voneinander Wasserstoff oder Alkyl mit 1,2,3 oder 4 C-Atomen;
oder
R(10) Heteroaryl mit 1,2,3,4,5,6,7,8 oder 9-C-Atomen, das unsubstituiert oder substituiert ist mit 1-3 Substituenten aus der Gruppe F, Cl, Br, I, CF₃, CH₃, Methoxy, Hydroxy oder NR(14)R(15);
R(14) und R(15) unabhängig voneinander Wasserstoff oder Alkyl mit 1,2,3 oder 4 C-Atomen;
oder
R(2) und R(4) gemeinsam eine zweite Bindung zwischen den die Reste R(3) und R(5) tragenden Kohlenstoffatomen, wobei R(1), R(3), R(5) wie oben definiert sind;
R(6) und R(7) unabhängig voneinander Wasserstoff, F, Cl, Br, I, CF₃, -C≡N, -NO₂, SOₚ-R(16),CO-R(23) oder O-R(24);
R(23) Wasserstoff, Alkyl mit 1,2,3,4,5,6,7 oder 8 C-Atomen oder OR(25);
R(25) Wasserstoff, Alkyl mit 1,2,3,4,5,6,7 oder 8 C-Atomen;
R(24) Wasserstoff, Alkyl mit 1,2,3,4,5,6,7 oder 8 C-Atomen oder Phenyl, das unsubstituiert oder substituiert ist mit 1-3 Substituenten aus der Gruppe F, Cl, Br, I, CF₃, Methyl, Methoxy, Hydroxy oder NR(28)R(29);
R(28) und R(29) H oder Alkyl mit 1,2,3 oder 4 C-Atomen;
R(16) Alkyl mit 1,2,3,4,5,6,7 oder 8 C-Atomen, Phenyl, das unsubstituiert oder substituiert ist mit 1-3 Substituenten aus der Gruppe F, Cl, Br, I, CF₃, Methyl, Methoxy, Hydroxy oder NR(26)R(27);
R(26) und R(27) gleich H oder Alkyl mit 1,2,3 oder 4 C-Atomen;
p Null, 1 oder 2;
sowie deren physiologisch verträglichen Salze.

2. Verbindung der Formel (I) gemäß Anspruch 1, in der bedeuten:
R(1) Wasserstoff, Alkyl mit 1,2,3 oder 4 C-Atomen, 1-Naphthyl, 2-Naphthyl, -CₐH₂ₐ-Cycloalkyl mit 3, 4, 5, 6 oder 7 C-Atomen oder -CₐH₂ₐ-Phenyl, wobei der Phenylteil unsubstituiert oder substituiert ist mit 1-2 Substituenten aus der Gruppe Alkyl mit 1,2,3 oder 4 C-Atomen, F, Cl, CF₃, SOₙR(11), OR(17), NR(8)R(9), -C≡N, oder CO-R(22);
R(11) Alkyl mit 1,2,3 oder 4 C-Atomen oder NR(20)R(21);
R(20) und R(21) unabhängig voneinander Wasserstoff, Methyl oder Ethyl;
R(17) Wasserstoff oder Alkyl mit 1,2,3 oder 4 C-Atomen;
R(8) und R(9) unabhängig voneinander Wasserstoff, Methyl oder Ethyl;
R(22) Wasserstoff, Alkyl mit 1,2,3 oder 4 C-Atomen oder OR(30);
R(30) Wasserstoff, Alkyl mit 1,2,3 oder 4 C-Atomen;
a Null oder 1;
n n Null oder 2;
oder
R(1) und R(3) gemeinsam mit dem sie tragenden C-Atom Cycloalkyl mit 3, 4, 5, 6 oder 7 C-Atomen oder Fluorenyl;
R(2) und R(4) unabhängig voneinander Wasserstoff oder F; oder
R(2) und R(4) gemeinsam eine zweite Bindung zwischen den die Reste R(3) und R(5) tragenden Kohlenstoffatomen;
R(3) und R(5) unabhängig voneinander Wasserstoff, F, CF₃, O-R(10), Alkyl mit 1,2,3 oder 4 C-Atomen, Cycloalkyl mit 3, 4, 5, 6 oder 7 C-Atomen, -C_{g}H_{2g}-Phenyl, wobei der Phenylteil unsubstituiert oder substituiert ist mit 1-2 Substituenten aus der Gruppe F, Cl, CF₃, Methyl, Methoxy, Hydroxy oder NR(18)R(19);
R(18) und R(19) unabhängig voneinander gleich Wasserstoff, Methyl oder Ethyl;
g Null oder 1 ;
R(10) Wasserstoff, Alkyl mit 1, 2, 3 oder 4 C-Atomen, Phenyl, das unsubstituiert oder substituiert ist mit 1-2 Substituenten aus der Gruppe F, Cl, CF₃, Methyl, Methoxy, Hydroxy oder NR(12)R(13);
R(12) und R(13) unabhängig voneinander gleich Wasserstoff, Methyl oder Ethyl;
oder
R(10) Heteroaryl mit 1, 2, 3, 4, 5, 6, 7, 8 oder 9 C-Atomen, das unsubstituiert oder substituiert ist mit 1-2 Substituenten aus der Gruppe F, Cl, CF₃, CH₃, Methoxy, Hydroxy oder NR(14)R(15);
R(14) and R(15) unabhängig voneinander Wasserstoff, Methyl oder Ethyl;
R(6) und R(7) unabhängig voneinander Wasserstoff, F, Cl, CF₃, -C≡N, SOₚ-R(16), CO-R(23) oder O-R(24);
R(23) Wasserstoff, Alkyl mit 1,2,3 oder 4 C-Atomen oder OR(25);
R(25) Wasserstoff, Alkyl mit 1,2,3 oder 4 C-Atomen;
R(24) Wasserstoff, Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(28)R(29);
R(28) und R(29) unabhängig voneinander Wasserstoff, Methyl oder Ethyl;
R(16) Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(26)R(27);
R(26) und R(27) unabhängig voneinander Wasserstoff, Methyl oder Ethyl;
p Null oder 2;
sowie deren physiologisch verträglichen Salze.

3. Verbindung der Formel (I) nach einem oder mehreren der Ansprüche 1 und 2, in der bedeuten:
R(1) Methyl, Ethyl, 1-Naphthyl, 2-Naphthyl,-CₐH₂ₐ-Cycloalkyl mit 3, 4, 5, 6 oder 7 C-Atomen oder -CₐH₂ₐ-Phenyl, wobei der Phenylteil unsubstituiert oder substituiert ist mit 1-2 Substituenten aus der Gruppe Alkyl mit 1,2,3 oder 4 C-Atomen, F, Cl, CF₃, SO₂R(11), OR(17), NR(8)R(9), -C≡N, oder CO-R(22);
R(11) Methyl oder Dimethylamino;
R(17) Wasserstoff, Methyl oder Ethyl;
R(8) und R(9) unabhängig voneinander Wasserstoff, Methyl oder Ethyl,
R(22) Wasserstoff oder Alkyl mit 1,2,3 oder 4 C-Atomen;
a Null oder 1;
oder
R(1) und R(3) gemeinsam mit dem sie tragenden C-Atom Cycloalkyl mit 3,4, 5, 6 oder 7 C-Atomen oder Fluorenyl;
R(2) und R(4) unabhängig voneinander Wasserstoff oder F; oder
R(2) und R(4) gemeinsam eine zweite Bindung zwischen den die Reste R(3) und R(5) tragenden Kohlenstoffatomen;
R(3) und R(5) unabhängig voneinander Wasserstoff, F, CF₃, O-R(10), Alkyl mit 1,2,3 oder 4 C-Atomen oder -C_{g}H_{2g}-Phenyl, das unsubstituiert oder substituiert ist mit 1-2 Substituenten aus der Gruppe F, Cl, CF₃, Methyl, Methoxy, Hydroxy oder NR(18)R(19);
R(18) und R(19) unabhängig voneinander Wasserstoff, Methyl oder Ethyl;
g Null oder 1;
R(10) Wasserstoff, Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(12)R(13);
R(12) und R(13) gleich Wasserstoff, Methyl oder Ethyl;
oder
R(10) Heteroaryl mit 1,2,3,4,5,6,7,8 oder 9 C-Atomen, das unsubstituiert oder substituiert ist mit 1-2 Substituenten aus der Gruppe F, Cl, CF₃, CH₃, Methoxy, Hydroxy oder Dimethylamino;
R(6) und R(7) unabhängig voneinander Wasserstoff, F, Cl, CF₃, -C≡N, SO₂-R(16), CO-R(23) oder O-R(24);
R(23) Wasserstoff oder Alkyl mit 1, 2, 3 oder 4 C-Atomen;
R(24) Wasserstoff, Alkyl mit 1,2,3 oder 4 C-Atomen oder Phenyl, das unsubstituiert oder substituiert ist mit 1-2 Substituenten aus der Gruppe F, Cl, CF₃, Methyl, Methoxy, Hydroxy oder NR(28)R(29);
R(28) und R(29) unabhängig voneinander Wasserstoff, Methyl oder Ethyl;
R(16) Alkyl mit 1,2,3 oder 4 C-Atomen;
sowie deren physiologisch verträglichen Salze.

4. Verbindung der Formel (I) gemäß einem oder mehreren der Ansprüche 1-3, in der bedeuten:
R(1) Methyl, Ethyl, 1-Naphthyl, 2-Naphthyl, Cycloalkyl mit 3, 4, 5, 6 oder 7 C-Atomen oder Phenyl, das unsubstituiert oder substituiert ist mit einem Substituenten aus der Gruppe Alkyl mit 1,2,3 oder 4 C-Atomen, F, Cl, CF₃, SO₂R(11), OR(17), NR(8)R(9), oder CO-R(22);
R(11) Methyl oder Dimethylamino;
R(17) Wasserstoff, Methyl oder Ethyl;
R(8) und R(9) unabhängig voneinander gleich Wasserstoff, Methyl oder Ethyl;
R(22) Wasserstoff oder Alkyl mit 1,2,3 oder 4 C-Atomen;
oder
R(1) und R(3) gemeinsam mit dem sie tragenden C-Atom Cycloalkyl mit 3, 4, 5, 6 oder 7 C-Atomen oder Fluorenyl;
R(2) und R(4) Wasserstoff; oder
R(2) und R(4) gemeinsam eine zweite Bindung zwischen den die Reste R(3) und R(5) tragenden Kohlenstoffatomen;
R(3) und R(5) unabhängig voneinander Wasserstoff, CF₃, O-R(10), Alkyl mit 1, 2, 3 oder 4 C-Atomen oder Phenyl, das unsubstituiert oder substituiert ist mit einem Substituenten aus der Gruppe F, Cl, CF₃, Methyl, Methoxy, Hydroxy oder NR(18)R(19);
R(18) und R(19) unabhängig voneinander Wasserstoff, Methyl oder Ethyl;
R(10) Wasserstoff, Alkyl mit 1,2,3 oder 4 C-Atomen oder Phenyl, das unsubstituiert oder substituiert ist mit 1 Substituenten aus der Gruppe F, Cl, CF₃, Methyl, Methoxy, Hydroxy oder NR(12)R(13);
R(12) und R(13) unabhängig voneinander Wasserstoff, Methyl oder Ethyl,
oder
R(10) Heteroaryl mit 1, 2, 3, 4, 5, 6, 7, 8 oder 9 C-Atomen, das unsubstituiert oder substituiert ist mit einem Substituenten aus der Gruppe F, Cl, CF₃, CH₃, Methoxy, Hydroxy oder Dimethylamino;
R(6) und R(7) unabhängig voneinander Wasserstoff, F, Cl, CF₃, SO₂-CH₃, CO-R(23) oder O-R(24);
R(23) Wasserstoff oder Alkyl mit 1,2,3 oder 4 C-Atomen;
R(24) Wasserstoff, Alkyl mit 1,2,3 oder 4 C-Atomen oder Phenyl, das unsubstituiert oder substituiert ist mit 1 Substituenten aus der Gruppe F, Cl, CF₃, Methyl, Methoxy, Hydroxy oder NR(28)R(29);
R(28) und R(29) unabhängig voneinander Wasserstoff, Methyl oder Ethyl;
sowie deren physiologisch verträglichen Salze.

5. Verwendung einer Verbindung der Formel I nach einem oder mehreren der Ansprüche 1-4 sowie von deren physiologisch verträglichen Salzen zur Herstellung eines Medikaments zur Behandlung oder Prophylaxe von durch ischämische Zustände hervorgerufenen Krankheiten oder zur Herstellung eines Medikaments zur Behandlung von gestörtem Atemantrieb.

6. Verwendung einer Verbindung der Formel I nach einem oder mehreren der Ansprüche 1-4 zur Herstellung eines Medikaments zur Behandlung oder Prophylaxe von durch ischämische Zustände hervorgerufenen Krankheiten.

7. Verwendung einer Verbindung der Formel I nach einem oder mehreren der Ansprüche 1-4 zur Herstellung eines Medikaments zur Behandlung oder Prophylaxe des Herzinfarkts.

8. Verwendung einer Verbindung der Formel I nach einem oder mehreren der Ansprüche 1-4 zur Herstellung eines Medikaments zur Behandlung oder Prophylaxe der Angina Pectoris.

9. Verwendung einer Verbindung der Formel I nach einem oder mehreren der Ansprüche 1-4 zur Herstellung eines Medikaments zur Behandlung oder Prophylaxe von ischämischen Zuständen des Herzens.

10. Verwendung einer Verbindung der Formel I nach einem oder mehreren der Ansprüche 1-4 zur Herstellung eines Medikaments zur Behandlung oder Prophylaxe von ischämischen Zuständen des peripheren und zentralen Nervensystems und des Schlaganfalls.

11. Verwendung einer Verbindung der Formel I nach einem oder mehreren der Ansprüche 1-4 zur Herstellung eines Medikaments zur Behandlung oder Prophylaxe von ischämischen Zuständen peripherer Organe und Gliedmaßen.

12. Verwendung einer Verbindung der Formel I nach einem oder mehreren der Ansprüche 1-4 zur Herstellung eines Medikaments zur Behandlung von Schockzuständen.

13. Verwendung einer Verbindung der Formel I nach einem oder mehreren der Ansprüche 1-4 zur Herstellung eines Medikaments zum Einsatz bei chirurgischen Operationen und Organtransplantationen.

14. Verwendung einer Verbindung der Formel I nach einem oder mehreren der Ansprüche 1-4 zur Herstellung eines Medikaments zur Konservierung und Lagerung von Transplantaten für chirurgische Maßnahmen.

15. Verwendung einer Verbindung der Formel I nach einem oder mehreren der Ansprüche 1-4 zur Herstellung eines Medikaments zur Behandlung von Krankheiten, bei denen die Zellproliferation eine primäre oder sekundäre Ursache darstellt.

16. Verwendung einer Verbindung der Formel I nach einem oder mehreren der Ansprüche 1-4 zur Herstellung eines Medikaments zur Behandlung oder Prophylaxe von gestörtem Atemantrieb.

17. Arzneimittel, **gekennzeichnet durch** einen wirksamen Gehalt einer Verbindung der Formel I nach einem oder mehreren der Ansprüche 1-4.

18. Arzneimittel gemäß Anspruch 17, **dadurch gekennzeichnet, daß** es zusätzlich eine wirksame Menge eines Natrium/Wasserstoff-Anstanscher (NHE)-Inhibitors und/oder eine wirksame Substanz aus einer anderen Herz-Kreislauf-Wirkstoffklasse, oder deren physiologisch verträglichen Salze enthält.

## Claims

1. A compound of the formula (I), 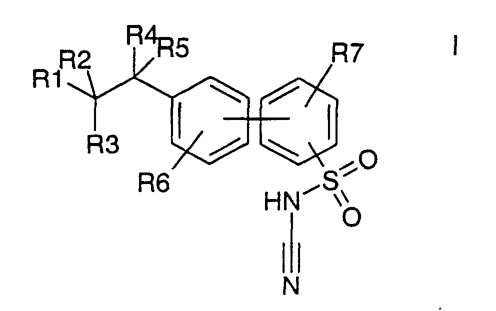 in which the symbols have the following meaning:
R(1) is hydrogen, alkyl having 1,2,3,4,5,6,7 or 8 carbon atoms, 1-naphthyl, 2-naphthyl, -CₐH₂ₐ-cycloalkyl having 3,4,5,6 or 7 carbon atoms or -CₐH₂ₐ-phenyl, where the phenyl moiety is unsubstituted or substituted by 1-3 substituents from the group consisting of alkyl having 1,2,3,4,5,6,7 or 8 carbon atoms, F, Cl, Br, I, CF₃, SOₙR(11), OR(17), NR(8)R(9), -C≡N, -NO₂ or CO-R(22);
R(11) is alkyl having 1,2,3 or 4 carbon atoms or NR(20)R(21);
R(20) and R(21) independently of one another are hydrogen or alkyl having 1,2,3 or 4 carbon atoms;
R(17) is hydrogen or alkyl having 1,2,3 or 4 carbon atoms;
R(8) and R(9) independently of one another are hydrogen or alkyl having 1,2,3 or 4 carbon atoms;
R(22) is hydrogen, alkyl having 1,2,3,4,5,6,7 or 8 carbon atoms or OR(30);
R(30) is hydrogen, alkyl having 1,2,3,4,5,6,7 or 8 carbon atoms;
a is zero, 1 or 2;
n is zero, 1 or 2;
or
R(1) and R(3) together with the carbon atom carrying them are cycloalkyl having 3,4,5,6 or 7 carbon atoms or fluorenyl;
R(2), R(3), R(4) and R(5) independently of one another are hydrogen, F, CF₃, O-R(10), alkyl having 1,2,3,4,5,6,7 or 8 carbon atoms, cycloalkyl having 3, 4, 5, 6 or 7 carbon atoms, -C_{g}H_{2g}-phenyl, where the phenyl moiety is unsubstituted or substituted by 1-3 substituents from the group consisting of F, Cl, Br, I, CF₃, methyl, methoxy, hydroxyl or NR(18)R(19);
R(18) and R(19) independently of one another are hydrogen or alkyl having 1,2,3 or 4 carbon atoms;
g is zero, 1 or 2;
R(10) is hydrogen, alkyl having 1,2,3,4,5,6,7 or 8 carbon atoms, phenyl which is unsubstituted or substituted by 1-3 substituents from the group consisting of F, Cl, Br, I, CF₃, methyl, methoxy, hydroxyl or NR(12)R(13);
R(12) and R(13) independently of one or alkyl another are hydrogen, methyl or ethyl;
or
R(10) is heteroaryl having 1,2,3,4,5,6,7,8 or 9 carbon atoms, which is unsubstituted or substituted by 1-3 substituents from the group consisting of F, Cl, Br, I, CF₃, CH₃, methoxy, hydroxyl or NR(14)R(15);
R(14) and R(15) independently of one another are hydrogen or alkyl having 1,2,3 or 4 carbon atoms;
or
R(2) and R(4) together are a second bond between the carbon atoms carrying the radicals R(3) and R(5), where R(1), R(3), R(5) are as defined above;
R(6) and R(7) independently of one another are hydrogen, F, Cl, Br, I, CF₃, -C≡N, -NO₂, SOₚ- R(16),CO-R(23) or O-R(24);
R(23) is hydrogen, alkyl having 1,2,3,4,5,6,7 or 8 carbon atoms or OR(25);
R(25) is hydrogen, alkyl having 1,2,3,4,5,6,7 or 8 carbon atoms;
R(24) is hydrogen, alkyl having 1,2,3,4,5,6,7 or 8 carbon atoms or phenyl, which is unsubstituted or substituted by 1-3 substituents from the group consisting of F, Cl, Br, I, CF₃, methyl, methoxy, hydroxyl or NR(28)R(29);
R(28) and R(29) are H or alkyl having 1,2,3 or 4 carbon atoms;
R(16) is alkyl having 1,2,3,4,5,6,7 or 8 carbon atoms, phenyl which is unsubstituted or substituted by 1-3 substituents from the group consisting of F, Cl, Br, I, CF₃, methyl, methoxy, hydroxyl or NR(26)R(27);
R(26) and R(27) are H or alkyl having 1,2,3 or 4 carbon atoms;
p is zero, 1 or 2;
or its physiologically tolerable salts.

2. A compound of the formula (I) as claimed in claim 1, in which:
R(1) is hydrogen, alkyl having 1,2,3 or 4 carbon atoms, 1-naphthyl, 2-naphthyl, -CₐH₂ₐ-cycloalkyl having 3, 4, 5, 6 or 7 carbon atoms or -CₐH₂ₐ-phenyl, where the phenyl moiety is unsubstituted or substituted by 1-2 substituents from the group consisting of alkyl having 1,2,3 or 4 carbon atoms, F, Cl, CF₃, SOₙR(11), OR(17), NR(8)R(9), -C≡N, or CO-R(22);
R(11) is alkyl having 1,2,3 or 4 carbon atoms or NR(20)R(21) ;
R(20) and R(21) independently of one another are hydrogen, methyl or ethyl;
R(17) is hydrogen or alkyl having 1,2,3 or 4 carbon atoms;
R(8) and R(9) independently of one another are hydrogen, methyl or ethyl;
R(22) is hydrogen, alkyl having 1,2,3 or 4 carbon atoms or OR(30);
R(30) is hydrogen, alkyl having 1,2,3 or 4 carbon atoms;
a is zero or 1;
n is zero or 2;
or
R(1) and R(3) together with the carbon atom carrying them are cycloalkyl having 3, 4, 5, 6 or 7 carbon atoms or fluorenyl;
R(2) and R(4) independently of one another are hydrogen or F; or
R(2) and R(4) together are a second bond between the carbon atoms carrying the radicals R(3) and R(5);
R(3) and R(5) independently of one another are hydrogen, F, CF₃, O-R(10), alkyl having 1,2,3 or 4 carbon atoms, cycloalkyl having 3, 4, 5, 6 or 7 carbon atoms, -C_{g}H_{2g}-phenyl, where the phenyl moiety is unsubstituted or substituted by 1-2 substituents from the group consisting of F, Cl, CF₃, methyl, methoxy, hydroxyl or NR(18)R(19);
R(18) and R(19) independently of one another are hydrogen, methyl or ethyl;
g is zero or 1 ;
R(10) is hydrogen, alkyl having 1, 2, 3 or 4 carbon atoms, phenyl which is unsubstituted or substituted by 1-2 substituents from the group consisting of F, Cl, CF₃, methyl, methoxy, hydroxyl or NR(12)R(13);
R(12) and R(13) independently of one another are hydrogen, methyl or ethyl;
or
R(10) is heteroaryl having 1, 2, 3, 4, 5, 6, 7, 8 or 9 carbon atoms, which is unsubstituted or substituted by 1-2 substituents from the group consisting of F, Cl, CF₃, CH₃, methoxy, hydroxyl or NR(14)R(15) ;
R(14) and R(15) independently of one another are hydrogen, methyl or ethyl;
R(6) and R(7) independently of one another are hydrogen, F, Cl, CF₃, -C≡N, SOₚ-R(16), CO-R(23) or O-R(24);
R(23) is hydrogen, alkyl having 1,2,3 or 4 carbon atoms or OR(25);
R(25) is hydrogen, alkyl having 1,2,3 or 4 carbon atoms;
R(24) is hydrogen, alkyl having 1,2,3 or 4 carbon atoms or phenyl which is unsubstituted or substituted by 1-2 substituents from the group consisting of F, Cl, CF₃, methyl, methoxy, hydroxyl or NR(28)R(29);
R(28) and R(29) independently of one another are hydrogen, methyl or ethyl;
R(16) is alkyl having 1,2,3 or 4 carbon atoms or phenyl which is unsubstituted or substituted by 1-2 substituents from the group consisting of F, Cl, CF₃, methyl, methoxy, hydroxyl or NR(26)R(27);
R(26) and R(27) independently of one another are hydrogen, methyl or ethyl;
p is zero or 2;
or its physiologically tolerable salts.

3. A compound of the formula (I) as claimed in one or more of claims 1 and 2, in which:
R(1) is methyl, ethyl, 1-naphthyl, 2-naphthyl, -CₐH₂ₐ-cycloalkyl having 3, 4, 5, 6 or 7 carbon atoms or -CₐH₂ₐ-phenyl, where the phenyl moiety is unsubstituted or substituted by 1-2 substituents from the group consisting of alkyl having 1,2,3 or 4 carbon atoms, F, Cl, CF₃, SO₂R(11), OR(17), NR(8)R(9), -C≡N, or CO-R(22);
R(11) is methyl or dimethylamino;
R(17) is hydrogen, methyl or ethyl;
R(8) and R(9) independently of one another are hydrogen, methyl or ethyl,
R(22) is hydrogen or alkyl having 1,2,3 or 4 carbon atoms;
a is zero or 1;
or
R(1) and R(3) together with the carbon atom carrying them are cycloalkyl having 3,4,5,6 or 7 carbon atoms or fluorenyl;
R(2) and R(4) independently of one another are hydrogen or F; or
R(2) and R(4) together are a second bond between the carbon atoms carrying the radicals R(3) and R(5);
R(3) and R(5) independently of one another are hydrogen, F, CF₃, O-R(10), alkyl having 1,2,3 or 4 carbon atoms or -C_{g}H_{2g}-phenyl which is unsubstituted or substituted by 1-2 substituents from the group consisting of F, Cl, CF₃, methyl, methoxy, hydroxyl or NR(18)R(19);
R(18) and R(19) independently of one another are hydrogen, methyl or ethyl;
g is zero or 1;
R(10) is hydrogen, alkyl having 1,2,3 or 4 carbon atoms or phenyl which is unsubstituted or substituted by 1-2 substituents from the group consisting of F, Cl, CF₃, methyl, methoxy, hydroxyl or NR(12)R(13);
R(12) and R(13) are hydrogen, methyl or ethyl;
or
R(10) is heteroaryl having 1,2,3,4,5,6,7,8 or 9 carbon atoms, which is unsubstituted or substituted by 1-2 substituents from the group consisting of F, Cl, CF₃, CH₃, methoxy, hydroxyl or dimethylamino;
R(6) and R(7) independently of one another are hydrogen, F, Cl, CF₃, -C≡N, SO₂-R(16), CO-R(23) or OR(24);
R(23) is hydrogen or alkyl having 1, 2, 3 or 4 carbon atoms;
R(24). is hydrogen, alkyl having 1,2,3 or 4 carbon atoms or phenyl which is unsubstituted or substituted by 1-2 substituents from the group consisting of F, Cl, CF₃, methyl, methoxy, hydroxyl or NR(28)R(29);
R(28) and R(29) independently of one another are hydrogen, methyl or ethyl;
R(16) is alkyl having 1,2,3 or 4 carbon atoms;
or its physiologically tolerable salts.

4. A compound of the formula (I) as claimed in one or more of claims 1-3, in which:
R(1) is methyl, ethyl, 1-naphthyl, 2-naphthyl, cycloalkyl having 3, 4, 5, 6 or 7 carbon atoms or phenyl which is unsubstituted or substituted by a substituent from the group consisting of alkyl having 1,2,3 or 4 carbon atoms, F, Cl, CF₃, SO₂R(11), OR(17), NR(8)R(9) or CO-R(22);
R(11) is methyl or dimethylamino;
R(17) is hydrogen, methyl or ethyl;
R(8) and R(9) independently of one another are hydrogen, methyl or ethyl;
R(22) is hydrogen or alkyl having 1,2,3 or 4 carbon atoms;
or
R(1) and R(3) together with the carbon atom carrying them are cycloalkyl having 3, 4, 5, 6 or 7 carbon atoms or fluorenyl;
R(2) and R(4) are hydrogen; or
R(2) and R(4) together are a second bond between the carbon atoms carrying the radicals R(3) and R(5);
R(3) and R(5) independently of one another are hydrogen, CF₃, O-R(10), alkyl having 1, 2, 3 or 4 carbon atoms or phenyl which is unsubstituted or substituted by a substituent from the group consisting of F, Cl, CF₃, methyl, methoxy, hydroxyl or NR(18)R(19);
R(18) and R(19) independently of one another are hydrogen, methyl or ethyl;
R(10) is hydrogen, alkyl having 1,2,3 or 4 carbon atoms or phenyl which is unsubstituted or substituted by 1 substituent from the group consisting of F, Cl, CF₃, methyl, methoxy, hydroxyl or NR(12)R(13);
R(12) and R(13) independently of one another are hydrogen, methyl or ethyl,
or
R(10) is heteroaryl having 1, 2, 3, 4, 5, 6, 7, 8 or 9 carbon atoms, which is unsubstituted or substituted by a substituent from the group consisting of F, Cl, CF₃, CH₃, methoxy, hydroxyl or dimethylamino;
R(6) and R(7) independently of one another are hydrogen, F, Cl, CF₃, SO₂-CH₃, CO-R(23) or 0R(24);
R(23) is hydrogen or alkyl having 1,2,3 or 4 carbon atoms;
R(24) is hydrogen, alkyl having 1,2,3 or 4 carbon atoms or phenyl which is unsubstituted or substituted by 1 substituent from the group consisting of F, Cl, CF₃, methyl, methoxy, hydroxyl or NR(28)R(29);
R(28) and R(29) independently of one another are hydrogen, methyl or ethyl;
or its physiologically tolerable salts.

5. The use of a compound of the formula I as claimed in one or more of claims 1-4 and of its physiologically tolerable salts for the production of a medicament for the treatment or prophylaxis of illnesses caused by ischemic conditions or for the production of a medicament for the treatment of impaired respiratory drive.

6. The use of a compound of the formula I as claimed in one or more of claims 1-4 for the production of a medicament for the treatment or prophylaxis of illnesses caused by ischemic conditions.

7. The use of a compound of the formula I as claimed in one or more of claims 1-4 for the production of a medicament for the treatment or prophylaxis of cardiac infarct.

8. The use of a compound of the formula I as claimed in one or more of claims 1-4 for the production of a medicament for the treatment or prophylaxis of angina pectoris.

9. The use of a compound of the formula I as claimed in one or more of claims 1-4 for the production of a medicament for the treatment or prophylaxis of ischemic conditions of the heart.

10. The use of a compound of the formula I as claimed in one or more of claims 1-4 for the production of a medicament for the treatment or prophylaxis of ischemic conditions of the peripheral and central nervous system and of stroke.

11. The use of a compound of the formula I as claimed in one or more of claims 1-4 for the production of a medicament for the treatment or prophylaxis of ischemic conditions of peripheral organs and limbs.

12. The use of a compound of the formula I as claimed in one or more of claims 1-4 for the production of a medicament for the treatment of states of shock.

13. The use of a compound of the formula I as claimed in one or more of claims 1-4 for the productioin of a medicament for use in surgical operations and organ transplantations.

14. The use of a compound of the formula I as claimed in one or more of claims 1-4 for the production of a medicament for the preservation and storage of transplants for surgical measures.

15. The use of a compound of the formula I as claimed in one or more of claims 1-4 for the production of a medicament for the treatment of illnesses in which cell proliferation is a primary or secondary cause.

16. The use of a compound of the formula I as claimed in one or more of claims 1-4 for the production of a medicament for the treatment or prophylaxis of impaired respiratory drive.

17. A pharmaceutical which comprises an efficacious amount of a compound of the formula I as claimed in one or more of claims 1-4.

18. A pharmaceutical as claimed in claim 17, which additionally contains an efficacious amount of a sodium/hydrogen exchanger (NHE) inhibitor and/or an active substance from another class of cardiovascular active compound, or its/their physiologically tolerable salts.

## Revendications

1. Composés de formule (I) 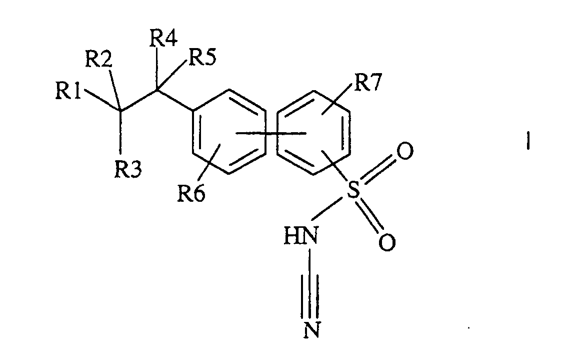 où les symboles ont la signification suivante :
R(1) représente l'hydrogène, alkyle ayant 1, 2; 3, 4, 5, 6, 7 ou 8 atomes de carbone, 1-naphtyle, 2-naphtyle, -CₐH₂ₐ-cycloalkyle ayant 3, 4, 5, 6 ou 7 atomes de carbone ou -CₐH₂ₐ-phényle, où la partie phényle est non substituée ou substituée par 1 à 3 substituants du groupe de alkyle ayant 1, 2, 3, 4, 5, 6, 7 ou 8 atomes de carbone, F, Cl, Br, I, CF₃, SOₙR(11), OR(17), NR(8)R(9), -C≡N, -NO₂ et CO-R(22) ;
R(11) représente alkyle ayant 1, 2, 3 ou 4 atomes de carbone ou NR(20)R(21) ;
R(20) et R(21) représentent indépendamment l'un de l'autre l'hydrogène ou alkyle ayant 1, 2, 3 ou 4 atomes de carbone ;
R(17) représente l'hydrogène ou alkyle ayant 1, 2, 3 ou 4 atomes de carbone ;
R(8) et R(9) représentent indépendamment l'un de l'autre l'hydrogène ou alkyle ayant 1, 2, 3 ou 4 atomes de carbone ;
R(22) représente l'hydrogène, alkyle ayant 1, 2, 3, 4, 5, 6, 7 ou 8 atomes de carbone ou OR(30);
R(30) représente l'hydrogène, alkyle ayant 1, 2, 3, 4, 5, 6, 7 ou 8 atomes de carbone ;
a représente 0, 1 ou 2 ;
n 1 représente 0, 1 ou 2 ;
ou bien
R(1) et R(3) représentent avec l'atome de carbone qui les porte cycloalkyle ayant 3, 4, 5, 6 ou 7 atomes de carbone ou fluorényle ;
R(2), R(3), R(4) et R(5) représentent indépendamment les uns des autres l'hydrogène, F, CF₃, O-R(10), alkyle ayant 1, 2, 3, 4, 5, 6, 7 ou 8 atomes de carbone, cycloalkyle ayant 3, 4, 5, 6 ou 7 atomes de carbone, -C_{g}H_{2g}-phényle, où la partie phényle est non substituée ou substituée par 1 à 3 substituants du groupe de F, Cl, Br, I, CF₃, méthyle, méthoxy, hydroxyle et NR(18)R(19) ;
R(18) et R(19) représentent indépendamment l'un de l'autre l'hydrogène ou alkyle ayant 1, 2, 3 ou 4 atomes de carbone ;
g représente 0, 1 ou 2 ;
R(10) représente l'hydrogène, alkyle ayant 1, 2, 3, 4, 5, 6, 7 ou 8 atomes de carbone, phényle qui est non substitué ou substitué par 1 à 3 substituants du groupe de F, Cl, Br, I, CF₃, méthyle, méthoxy, hydroxyle et NR(12)R(13) ;
R(12) et R(13) représentent indépendamment l'un de l'autre l'hydrogène ou alkyle ayant 1, 2, 3 ou 4 atomes de carbone ; ou bien
R(10) représente hétéroaryle ayant 1, 2, 3, 4, 5, 6, 7, 8 ou 9 atomes de carbone, qui est non substitué ou substitué par 1 à 3 substituants du groupe de F, Cl, Br, I, CF₃, CH₃, méthoxy, hydroxyle et NR(14)R(15);
R(14) et R(15) représentent indépendamment l'un de l'autre l'hydrogène ou alkyle ayant 1, 2, 3 ou 4 atomes de carbone ; ou bien
R(2) et R(4) représentent ensemble une seconde liaison entre les atomes de carbone portant les restes R(3) et R(5), où R(1), R(3) et R(5) sont définis comme ci-dessus ;
R(6) et R(7) représentent indépendamment l'un de l'autre l'hydrogène, F, Cl, Br, I, CF₃, -C≡N, -NO₂, SOₚ-R(16), CO-R(23) ou O-R(24) ;
R(23) représente l'hydrogène, alkyle ayant 1, 2, 3, 4, 5, 6, 7 ou 8 atomes de carbone ou OR(25):
R(25) représente l'hydrogène, alkyle ayant 1, 2, 3, 4, 5, 6, 7 ou 8 atomes de carbone;
R(24) représente l'hydrogène, alkyle ayant 1, 2, 3, 4, 5, 6, 7 ou 8 atomes de carbone ou phényle, qui est non substitué ou substitué par 1 à 3 substituants du groupe de F, Cl, Br, I, CF₃, méthyle, méthoxy, hydroxyle et NR(28)R(29) ;
R(28) et R(29) représentent H ou alkyle ayant 1, 2, 3 ou 4 atomes de carbone ;
R(16) représente alkyle ayant 1, 2, 3, 4, 5, 6, 7 ou 8 atomes de carbone, phényle, qui est non substitué ou substitué par 1 à 3 substituants du groupe de F, Cl, Br, I, CF₃, méthyle, méthoxy, hydroxyle et NR(26)R(27) ;
R(26) et R(27) représentent H ou alkyle ayant 1, 2, 3 ou 4 atomes de carbone ;
p représente 0, 1 ou 2 ;
ainsi que leurs sels physiologiquement acceptables.

2. Composé de formule (I) selon la revendication 1, où :
R(1) représente l'hydrogène, alkyle ayant 1, 2, 3 ou 4 atomes de carbone, 1-naphtyle, 2-naphtyle, -CₐH₂ₐ-cycloalkyle ayant 3, 4, 5, 6 ou 7 atomes de carbone ou -CₐH₂ₐ-phényle, où la partie phényle est non substituée ou substituée par 1 à 2 substituants du groupe de alkyle ayant 1, 2, 3 ou 4 atomes de carbone, F, Cl, CF₃, SOₙR(11), OR(17), NR(8)R(9), -C≡N et CO-R(22) ;
R(11) représente alkyle ayant 1, 2, 3 ou 4 atomes de carbone ou NR(20)R(21) ;
R(20) et R(21) représentent indépendamment l'un de l'autre l'hydrogène, méthyle ou éthyle ;
R(17) représente l'hydrogène ou alkyle ayant 1, 2, 3 ou 4 atomes de carbone ;
R(8) et R(9) représentent indépendamment l'un de l'autre l'hydrogène, méthyle ou éthyle ;
R(22) représente l'hydrogène, alkyle ayant 1, 2, 3 ou 4 atomes de carbone ou OR(30) ;
R(30) représente l'hydrogène, alkyle ayant 1, 2, 3 ou 4 atomes de carbone ;
a représente 0 ou 1 ;
n représente 0 ou 2 ;
ou bien
R(1) et R(3) représentent avec l'atome de carbone qui les porte cycloalkyle ayant 3, 4, 5, 6 ou 7 atomes de carbone ou fluorényle ;
R(2) et R(4) représentent indépendamment l'un de l'autre l'hydrogène ou F ; ou bien
R(2) et R(4) représentent ensemble une seconde liaison entre les atomes de carbone portant les restes R(3) et (R(5) ;
R(3) et R(5) représentent indépendamment l'un de l'autre l'hydrogène, F, CF₃, O-R(10), alkyle ayant 1, 2, 3 ou 4 atomes de carbone, cycloalkyle ayant 3, 4, 5, 6 ou 7 atomes de carbone, -C_{g}H_{2g}-phényle, où la partie phényle est non substituée ou substituée par 1 à 2 substituants du groupe de F, Cl, CF₃, méthyle, méthoxy, hydroxyle et NR(18)R(19) ;
R(18) et R(19) représentent indépendamment l'un de l'autre l'hydrogène, méthyle ou éthyle ;
g représente 0 ou 1 ;
R(10) représente l'hydrogène, alkyle ayant 1, 2, 3 ou 4 atomes de carbone, phényle qui est non substitué ou substitué par 1 à 2 substituants du groupe de F, Cl, CF₃, méthyle, méthoxy, hydroxyle et NR(12)R(13) ;
R(12) et R(13) représentent indépendamment l'un de l'autre l'hydrogène, méthyle ou éthyle ; ou bien
R(10) représente hétéroaryle ayant 1, 2, 3, 4, 5, 6, 7, 8 ou 9 atomes de carbone, qui est non substitué ou substitué par 1 à 2 substituants du groupe de F, Cl, CF₃, CH₃, méthoxy, hydroxyle et NR(14)R(15) ;
R(14) et R(15) représentent indépendamment l'un de l'autre l'hydrogène, méthyle ou éthyle ;
R(6) et R(7) représentent indépendamment l'un de l'autre l'hydrogène, F, Cl, CF₃, -C≡N, SOₚ-R(16), CO-R(23) et O-R(24) ;
R(23) représente l'hydrogène, alkyle ayant 1, 2, 3 ou 4 atomes de carbone ou OR(25);
R(25) représente l'hydrogène, alkyle ayant 1, 2, 3 ou 4 atomes de carbone ;
R(24) représente l'hydrogène, alkyle ayant 1, 2, 3 ou 4 atomes de carbone ou phényle, qui est non substitué ou substitué par 1 à 2 substituants du groupe de F, Cl, CF₃, méthyle, méthoxy, hydroxyle et NR(28)R(29) ;
R(28) et R(29) représentent indépendamment l'un de l'autre l'hydrogène, méthyle ou éthyle ;
R(16) représente alkyle ayant 1, 2, 3 ou 4 atomes de carbone ou phényle, qui est non substitué ou substitué par 1 à 2 substituants du groupe de F, Cl, CF₃, méthyle, méthoxy, hydroxyle et NR(26)R(27) ;
R(26) et R(27) représentent indépendamment l'un de l'autre l'hydrogène, méthyle ou éthyle ;
p représente 0 ou 2 ;
ainsi que ses sels physiologiquement acceptables.

3. Composé de formule (I) selon une ou plusieurs des revendications 1 et 2, où :
R(1) représente méthyle, éthyle, 1-naphtyle, 2-naphtyle, -CₐH₂ₐ-cycloalkyle ayant 3, 4, 5, 6 ou 7 atomes de carbone ou -CₐH₂ₐ-phényle, où la partie phényle est non substituée ou substituée par 1 à 2 substituants du groupe de alkyle ayant 1, 2, 3 ou 4 atomes de carbone, F, Cl, CF₃, SO₂R(11), OR(17), NR(8)R(9), -C≡N et CO-R(22) ;
R(11) représente méthyle ou diméthylamino ;
R(17) représente l'hydrogène, méthyle ou éthyle ;
R(8) et R(9) représentent indépendamment l'un de l'autre l'hydrogène, méthyle ou éthyle ;
R(22) représente l'hydrogène ou alkyle ayant 1, 2, 3 ou 4 atomes de carbone ;
a représente 0 ou 1 ;
ou bien
R(1) et R(3) représentent avec l'atome de carbone qui les porte cycloalkyle ayant 3, 4, 5, 6 ou 7 atomes de carbone ou fluorényle ;
R(2) et R(4) représentent indépendamment l'un de l'autre l'hydrogène ou F ; ou bien
R(2) et R(4) représentent ensemble une seconde liaison entre les atomes de carbone portant les restes R(3) et R(5) ;
R(3) et R(5) représentent indépendamment l'un de l'autre l'hydrogène, F, CF₃, O-R(10), alkyle ayant 1, 2, 3 ou 4 atomes de carbone ou -C_{g}H_{2g}-phényle qui est non substitué ou substitué par 1 à 2 substituants du groupe de F, Cl, CF₃, méthyle, méthoxy, hydroxyle et NR(18)R(19) ;
R(18) et R(19) représentent indépendamment l'un de l'autre l'hydrogène, méthyle ou éthyle ;
g représente 0 ou 1 ;
R(10) représente l'hydrogène, alkyle ayant 1, 2, 3 ou 4 atomes de carbone ou phényle qui est non substitué ou substitué par 1 à 2 substituants du groupe de F, Cl, CF₃, méthyle, méthoxy, hydroxyle et NR(12)R(13) ;
R(12) et R(13) représentent l'hydrogène, méthyle ou éthyle ; ou bien
R(10) représente hétéroaryle ayant 1, 2, 3, 4, 5, 6, 7, 8 ou 9 atomes de carbone, qui est non substitué ou substitué par 1 à 2 substituants du groupe de F, Cl, CF₃, CH₃, méthoxy, hydroxyle et diméthylamino ;
R(6) et R(7) représentent indépendamment de l'autre l'hydrogène, F, Cl, CF₃, -C≡N, SO₂-R(16), CO-R(23) et O-R(24) ;
R(23) représente l'hydrogène ou alkyle ayant 1, 2, 3 ou 4 atomes de carbone;
R(24) représente l'hydrogène, alkyle ayant 1, 2, 3 ou 4 atomes de carbone ou phényle, qui est non substitué ou substitué par 1 à 2 substituants du groupe de F, Cl, CF₃, méthyle, méthoxy, hydroxyle et NR(28)R(29) ;
R(28) et R(29) représentent indépendamment l'un de l'autre l'hydrogène, méthyle ou éthyle ;
R(16) représente alkyle ayant 1, 2, 3 ou 4 atomes de carbone ;
ainsi que ses sels physiologiquement acceptables.

4. Composé de formule (I) selon une ou plusieurs des revendications 1 à 3, où :
R(1) représente méthyle, éthyle, 1-naphtyle, 2-naphtyle, cycloalkyle ayant 3, 4, 5, 6 ou 7 atomes de carbone ou phényle qui est non substitué ou substitué par un substituant du groupe de alkyle ayant 1, 2, 3 ou 4 atomes de carbone, F, Cl, CF₃, SO₂R(11), OR(17), NR(8)R(9) et CO-R(22) ;
R(11) représente méthyle ou diméthylamino ;
R(17) représente l'hydrogène, méthyle ou éthyle ;
R(8) et R(9) représentent indépendamment l'un de l'autre l'hydrogène, méthyle ou éthyle ;
R(22) représente l'hydrogène ou alkyle ayant 1, 2, 3 ou 4 atomes de carbone ;
ou bien
R(1) et R(3) représentent avec l'atome de carbone qui les porte cycloalkyle ayant 3, 4, 5, 6 ou 7 atomes de carbone ou fluorényle ;
R(2) et R(4) représentent l'hydrogène ; ou bien
R(2) et R(4) représentent ensemble une seconde liaison entre les atomes de carbone portant les restes R(3) et (R(5) ;
R(3) et R(5) représentent indépendamment l'un de l'autre l'hydrogène, CF₃, O-R(10), alkyle ayant 1, 2, 3 ou 4 atomes de carbone ou phényle qui est non substitué ou substitué par un substituant du groupe de F, Cl, CF₃, méthyle, méthoxy, hydroxyle et NR(18)R(19) ;
R(18) et R(19) représentent indépendamment l'un de l'autre l'hydrogène, méthyle ou éthyle ;
R(10) représente l'hydrogène, alkyle ayant 1, 2, 3 ou 4 atomes de carbone ou phényle qui est non substitué ou substitué par 1 substituant du groupe de F, Cl, CF₃, méthyle, méthoxy, hydroxyle et NR(12)R(13) ;
R(12) et R(13) représentent indépendamment l'un de l'autre l'hydrogène, méthyle ou éthyle ;
ou bien
R(10) représente hétéroaryle ayant 1, 2, 3, 4, 5, 6, 7, 8 ou 9 atomes de carbone qui est non substitué ou substitué par un substituant du groupe de F, Cl, CF₃, CH₃, méthoxy, hydroxyle et diméthylamino ;
R(6) et R(7) représentent indépendamment l'un de l'autre l'hydrogène, F, Cl, CF₃, SO₂-CH₃, CO-R(23) ou O-R(24) ;
R(23) représente l'hydrogène ou alkyle ayant 1, 2, 3 ou 4 atomes de carbone ;
R(24) représente l'hydrogène, alkyle ayant 1, 2, 3 ou 4 atomes de carbone
ou phényle qui est non substitué ou substitué par 1 substituant du groupe de F, Cl, CF₃, méthyle, méthoxy, hydroxyle et NR(28)R(29) ;
R(28) et R(29) représentent indépendamment l'un de l'autre l'hydrogène, méthyle ou éthyle ;
ainsi que ses sels physiologiquement acceptables.

5. Utilisation d'un composé de formule I selon une ou plusieurs des revendications 1 à 4 ainsi que de ses sels physiologiquement acceptables pour la fabrication d'un médicament pour le traitement ou la prophylaxie de maladies provoquées par des états ischémiques ou pour la fabrication d'un médicament pour le traitement de l'impulsion respiratoire perturbée.

6. Utilisation d'un composé de formule I selon une ou plusieurs des revendications 1 à 4 pour la fabrication d'un médicament pour le traitement ou la prophylaxie de maladies provoquées par des états ischémiques.

7. Utilisation d'un composé de formule I selon une ou plusieurs des revendications 1 à 4 pour la fabrication d'un médicament pour le traitement ou la prophylaxie de l'infarctus du myocarde.

8. Utilisation d'un composé de formule I selon une ou plusieurs des revendications 1 à 4 pour la fabrication d'un médicament pour le traitement ou la prophylaxie de l'angine de poitrine.

9. Utilisation d'un composé de formule I selon une ou plusieurs des revendications 1 à 4 pour la fabrication d'un médicament pour le traitement ou la prophylaxie d'états ischémiques du coeur.

10. Utilisation d'un composé de formule I selon une ou plusieurs des revendications 1 à 4 pour la fabrication d'un médicament pour le traitement ou la prophylaxie d'états ischémiques du système nerveux périphérique et central et de l'apoplexie cérébrale.

11. Utilisation d'un composé de formule I selon une ou plusieurs des revendications 1 à 4 pour la fabrication d'un médicament pour le traitement ou la prophylaxie d'états ischémiques d'organes périphériques et de membres.

12. Utilisation d'un composé de formule I selon une ou plusieurs des revendications 1 à 4 pour la fabrication d'un médicament pour le traitement des états de choc.

13. Utilisation d'un composé de formule I selon une ou plusieurs des revendications 1 à 4 pour la fabrication d'un médicament destiné à être utilisé dans les opérations chirurgicales et les greffes d'organes.

14. Utilisation d'un composé de formule I selon une ou plusieurs des revendications 1 à 4 pour la fabrication d'un médicament pour la conservation et le stockage de greffes pour des opérations chirurgicales.

15. Utilisation d'un composé de formule I selon une ou plusieurs des revendications 1 à 4 pour la fabrication d'un médicament pour le traitement de maladies pour lesquelles la prolifération cellulaire constitue une cause primaire ou secondaire.

16. Utilisation d'un composé de formule I selon une ou plusieurs des revendications 1 à 4 pour la fabrication d'un médicament pour le traitement ou la prophylaxie de l'impulsion respiratoire perturbée.

17. Médicament **caractérisé par** une teneur efficace en un composé de formule I selon une ou plusieurs des revendications 1 à 4.

18. Médicament selon la revendication 17, **caractérisé en ce qu'**il contient en outre une quantité efficace d'un inhibiteur d'échangeur sodium/ hydrogène (NHE) et/ou une substance efficace d'une autre classe de principes actifs pour la circulation cardiaque, ou de leurs sels physiologiquement acceptables.
